# EUROPEAN PATENT SPECIFICATION

(11) **EP 3 890 781 B1**
(45) Date of publication and mention of the grant of the patent: **08.10.2025**
(21) Application number: 19820858.9
(22) Date of filing: 05.12.2019
(51) Int. Cl.: A61K 41/00, A61K 47/69, A61P 35/00, C07K 16/00, A61K 39/00, A61K 31/00, A61K 39/39, C07K 16/28, A61K 45/06, A61K 39/395

(54) **IMMUNE CHECKPOINT INHIBITOR IN COMBINATION WITH SONODYNAMIC THERAPY**
IMMUNCHECKPOINT-INHIBITOR IN KOMBINATION MIT SONODYNAMISCHER THERAPIE
INHIBITEUR DE POINT DE CONTRÔLE IMMUNITAIRE EN COMBINAISON AVEC LA THÉRAPIE SONODYNAMIQUE

(30) Priority: 05.12.2018 GB 201819853
(43) Date of publication of application: 13.10.2021
(73) Proprietor: SonoTarg Limited, Belfast BT1 4LS (GB)
(72) Inventor: CALLAN, John, Newtownabbey, Antrim BT37 0QB (GB); MCHALE, Anthony, Newtownabbey, Antrim BT37 0QB (GB)
(74) Representative: Dehns
(86) International application number: PCT/GB2019/053441
(87) International publication number: WO 2020/115491

(56) References cited:
- WO-A1-2018/220376
- NESBITT HEATHER ET AL: "Sonodynamic therapy complements PD-L1 immune checkpoint inhibition in a murine model of pancreatic cancer", CANCER LETTERS, NEW YORK, NY, US, vol. 517, 10 June 2021 (2021-06-10), pages 88 - 95, XP086706084, ISSN: 0304-3835, [retrieved on 20210610], DOI: 10.1016/J.CANLET.2021.06.003
- SHIYA ZHENG ET AL: "Targeted microbubbles with ultrasound irradiation and PD-1 inhibitor to increase antitumor activity in B-cell lymphoma", NANOMEDICINE, vol. 13, no. 3, 1 February 2018 (2018-02-01), GB, pages 297 - 311, XP055667366, ISSN: 1743-5889, DOI: 10.2217/nnm-2017-0296
- CONOR MCEWAN ET AL: "Combined sonodynamic and antimetabolite therapy for the improved treatment of pancreatic cancer using oxygen loaded microbubbles as a delivery vehicle", BIOMATERIALS., vol. 80, 1 February 2016 (2016-02-01), GB, pages 20 - 32, XP055349914, ISSN: 0142-9612, DOI: 10.1016/j.biomaterials.2015.11.033
- KRISTIAN M. HARGADON ET AL: "Immune checkpoint blockade therapy for cancer: An overview of FDA-approved immune checkpoint inhibitors", INTERNATIONAL IMMUNOPHARMACOLOGY, vol. 62, 1 September 2018 (2018-09-01), NL, pages 29 - 39, XP055637409, ISSN: 1567-5769, DOI: 10.1016/j.intimp.2018.06.001
- LOGAN KEIRAN ET AL: "Targeted chemo-sonodynamic therapy treatment of breast tumours using ultrasound responsive microbubbles loaded with paclitaxel, doxorubicin and Rose Bengal", EUROPEAN JOURNAL OF PHARMACEUTICS AND BIOPHARMACEUTICS, vol. 139, 5 April 2019 (2019-04-05), pages 224 - 231, XP085673751, ISSN: 0939-6411, DOI: 10.1016/J.EJPB.2019.04.003

## Description

### Technical field

The present invention relates generally to sonodynamic therapy and, more specifically, to such therapy for the treatment of deeply-sited tumours and associated metastatic disease in combination with immune checkpoint inhibitors. In particular, it relates to the treatment of pancreatic cancer (e.g. pancreatic ductal adenocarcinoma) and associated metastasis.

### Background of the invention

Conventional treatment of deeply-sited tumours typically involves major surgery, chemotherapy, radiotherapy or combinations of all of these. All three interventions may result in various complications including sepsis. Therefore, the development of more targeted and less invasive therapeutic approaches with improved efficacy to treat such patients is highly sought after. Pancreatic cancer is one example of a deeply-sited tumour. It remains one of the most lethal types of cancer known with less than 20% of those diagnosed being eligible for curative surgical treatment. It accounts for approximately 2% of all cancers with a five year survival of 15-21% in patients who have a surgical resection followed by systemic chemotherapy.

Methods known for use in the treatment of cancer include photodynamic therapy (PDT). PDT involves the application of photosensitising agents to the affected area, followed by exposure to photoactivating light to convert these into cytotoxic form. This results in the destruction of cells and surrounding vasculature in a target tissue. Photosensitisers which are currently approved for use in PDT absorb light in the visible region (below 700 nm). However, light of this wavelength has limited ability to penetrate the skin; this penetrates to a surface depth of only a few mm. Whilst PDT may be used to treat deeper sited target cells, this generally involves the use of a device, such as a catheter-directed fibre optic, for activation of the photosensitiser. Not only is this a complicated procedure, but it precludes access to certain areas of the body. It also compromises the non-invasive nature of the treatment. Thus, although appropriate for treating superficial tumours, the use of PDT in treating deeply seated cells, such as tumour masses, and anatomically less accessible lesions is limited.

Sonodynamic therapy (SDT) is a more recent concept and involves the combination of ultrasound and a sonosensitising drug (also referred to herein as a "sonosensitiser" or "sonosensitising agent"). In a manner similar to PDT, activation of the sonosensitiser by acoustic energy results in the generation of reactive oxygen species (ROS), such as singlet oxygen, at the target site of interest. Such species are cytotoxic, thereby killing the target cells or at least diminishing their proliferative potential. Many known photosensitising agents can be activated by acoustic energy and are thus suitable for use in SDT. Since ultrasound readily propagates through several cm of tissue, SDT provides a means by which tumours which are located deep within the tissues may be treated. As with light, ultrasound energy can also be focused on a tumour mass in order to activate the sonosensitiser thereby restricting its effects to the target site. SDT offers some significant advantages over PDT: ultrasound is widely accepted as a cost effective and safe clinical imaging modality and, unlike light, can be tightly focused with penetration in soft tissue up to several tens of centimetres depending on the ultrasound frequency used.

In WO 2012/143739 sonosensitisers are conjugated to a gas-filled microbubble (MB) to provide a microbubble-sonosensitiser "complex" (or "conjugate") for use in SDT. These complexes permit effective delivery of the active sonosensitiser in a site-specific manner by a controlled destruction of the bubble using ultrasound. Subsequent or simultaneous sono-activation of the targeted sonosensitiser results in cell destruction at the target site and regression of tumour tissues. The effectiveness of SDT using such complexes for the treatment of pancreatic cancer has been demonstrated in a pre-clinical mouse model bearing human xenograft BxPC-3 tumours (see McEwan et al., J Control Release. 2015; 203, 51-6). In a development of this work, a combined SDT / anti-metabolite therapy is proposed in WO 2017/089800 and also reported in McEwan et al., Biomaterials 2016; 80, 20-32.

The targeted delivery of a sonosensitiser and an anti-metabolite (e.g. 5-fluorouracil (5-FU) or gemcitabine) using a microbubble - either in the form of a single microbubble carrying both agents or separate microbubbles carrying the different agents - permits effective delivery of both agents to the tumour. Sono-activation of the targeted sonosensitiser results in the generation of ROS which destroy tumour cells at the target site. This action is complimented by the action of the anti-metabolite which exerts its cytotoxic effect directly at the target site. By using a microbubble as a carrier for both agents, non-specific uptake of these by target tissues is reduced. Such therapy thus provides a more targeted approach with improved efficacy and reduced side-effects compared to systemic administration of the anti-metabolite drug alone.

Many cancers still remain largely incurable. At least in part this is due to a step change from localised to metastatic disease in which cancer cells spread throughout the body. Tumours have also evolved to evade the body's own immune system. Immunotherapy represents an exciting development in the treatment of cancer and involves stimulating or priming a patient's immune system to seek out and destroy cancer cells. One class of immunotherapy is the use of immune checkpoint inhibitors which have been shown to be effective in treating certain cancers such as melanoma and lung cancer. However, their effect in pancreatic cancer is poor.

In clinical trials, immune checkpoint blockade has shown little benefit in the treatment of pancreatic ductal adenocarcinoma (PDAC) (see Morrison et al., Trends in Cancer (2018) 4: 418-428). Studies carried out by the inventors and documented herein confirm this finding. Two main reasons have been suggested for the poor effect of immune checkpoint inhibitors in pancreatic cancer: (i) pancreatic tumours are characterised by a highly immunosuppressive tumour microenvironment meaning that a low amount of cancer-fighting immune cells are produced; and (ii) pancreatic tumours have a dense protective coating called a "stroma" that acts as a barrier to entry for the cancer-fighting immune cells. To enhance the action of immunotherapy in pancreatic cancer it may therefore be beneficial to increase the number of cancer-fighting immune cells and/or improve their ability to infiltrate the tumour.

Abscopal responses in which a localised chemotherapeutic treatment stimulates the immune system and modulates the systemic regression of distant (e.g. metastatic) cancers have been observed, for example when carrying out radiotherapy. Such responses can be triggered more effectively when combining the cancer treatment (e.g. radiotherapy) with immunotherapy. However, relatively little is known about the abscopal response in SDT.

The effect of SDT using HPD (HiPorfin) on the induction of a systemic immune response has been investigated in liver cancer cell lines (see Zhang et al., Cancer Science 2018: 1-16). However, it cannot be predicted whether the abscopal effects seen in this tumour model would extend to other tumours, especially to pancreatic tumours due to the challenges associated with its treatment, for example as evidenced by the well-recognised ability of its stroma to protect the cancer cells from attack by the immune system and the current failure of existing immunotherapy in treating pancreatic cancer (see Sun et al., Ther. Clin. Risk Manag. (2018) 14: 1691-1700). Furthermore, the generation of "synergy" between any chemotherapy treatment and immunotherapy required to provide local treatment of tumours and induce an abscopal effect is inherently unpredictable due to the many different factors affecting the tumour immune interaction, e.g. chemotherapy-induced immunosuppression.

A need thus still exists for alternative (e.g. improved) methods for the treatment of deeply-sited, inaccessible tumours and metastases derived therefrom. In particular, a need exists for such methods for the treatment of metastatic pancreatic cancer.

### Summary of the invention

The inventors now propose that microbubble-delivered SDT is capable of improving T-cell infiltration into the tumour microenvironment to sensitise PDAC to checkpoint blockade thereby inducing a potent and specific anti-tumour immune response. Using a bilateral tumour model comprising KPC allografts in immunocompetent mice, the inventors have demonstrated that treating a target tumour ("primary tumour") with ultrasound targeted microbubble destruction (UTMD) mediated SDT produces an anti-tumour response in the distant (untreated or "off-target") tumour. Use of an immune checkpoint inhibitor, anti-PDL-1, further improves the SDT-mediated anti-tumour effect at the off-target tumour. A significant increase in cytotoxic T-cells is also observed in off-target tumours when the primary tumour is treated with SDT. Combined, these results suggest that microbubble-delivered SDT induces an "abscopal" effect that when combined with anti-PDL-1 further improves the reduction in tumour volume observed at the off-target tumour.

In view of these findings, the inventors propose various improvements in and relating to microbubble-delivered SDT. Specifically, the inventors propose that such therapy may be used not only in the targeted treatment of a primary tumour, but in view of the potential of this treatment to initiate an "abscopal" effect, they propose its extended use in the treatment of non-targeted tumours, e.g. in the treatment of metastatic disease or circulating tumour cells (CTCs), and in the treatment of other non-targeted primary tumours. The inventors' findings also offer the potential of additive effects, or even synergy, of microbubble-delivered SDT with immunotherapy-based strategies and so they now propose the treatment of tumours (both primary and metastatic tumours) using microbubble-delivered SDT in combination with immune checkpoint inhibitors.

The inventors further propose that such treatment models can be extended to the use of chemotherapy-SDT in which microbubbles are also used to deliver a chemotherapeutic agent to the targeted tumour (i.e. the primary tumour). Since this treatment involves targeted chemotherapy, exposure of normal tissue to cytotoxic chemotherapeutic drugs is reduced. Low circulating levels of chemotherapeutic drug permits survival of circulating immune cells thereby offering a better chance of exploiting the immune system in addressing both primary tumours and metastatic disease. The immuno-stimulatory effect of chemotherapy-SDT and the stromal modulation due to MB cavitation further facilitates the infiltration of immune checkpoint inhibitors and T-cells. Thus, the inventors also propose the use of microbubbles to target delivery of both chemotherapy and sonodynamic therapy to tumours and sensitise the cancer cells to attack by immunotherapy using immune checkpoint inhibitors. Since chemotherapy can provide some degree of immune stimulation in the treatment of cancer, this offers the potential of synergy with immunotherapy-based strategies, such as the combination with immune checkpoint inhibitors.

These proposals are considered to be of particular benefit in the context of treating pancreatic cancer. In particular, these provide a minimally invasive and highly focused treatment with the ability to significantly reduce tumour burden in pancreatic cancer. These are also expected to provide significant benefits in terms of improved survival rates for patients with pancreatic cancer and a better quality of life during treatment (due to the reduction in side-effects when compared to current standard of care drug based treatments).

The invention is as defined in the appended claims.

Any reference herein to methods of treatment of a human or non-human animal body by therapy are to be interpreted as references to the compounds, compositions and products of the present invention for use in such methods. Methods of medical treatment do not form part of the invention.

### Detailed description of the invention

### Definitions

As used herein, the term "microbubble" is intended to refer to a microsphere comprising a shell having an approximately spherical shape and which surrounds an internal void which comprises a gas or mixture of gases. The "shell" refers to the membrane which surrounds the internal void of the microbubble.

The terms "sonosensitiser", "sonosensitising agent" and "sonosensitising drug" are used interchangeably herein and are intended to refer to any compound which is capable of converting acoustic energy (e.g. ultrasound) into reactive oxygen species (ROS), such as singlet oxygen, that results in cell toxicity. In the microbubble-sonosensitiser complex for use in the invention, the sonosensitiser is Rose Bengal.

As used herein, the terms "sonodynamic therapy" and "sonodynamic treatment" are intended to refer to a method involving the combination of ultrasound and a sonosensitising agent in which activation of the sonosensitising agent by acoustic energy results in the generation of reactive oxygen species, such as singlet oxygen.

Immune checkpoints are known in the art and the term is well understood in the context of cancer therapy. Perhaps the most well-known are PD-1 and its ligand PDL-1, and CTLA-4. Others include OX40, TIM-3, KIR, LAG-3, VISTA and BTLA. Inhibitors of immune checkpoints, herein referred to as "immune checkpoint inhibitors", inhibit their normal immunosuppressive function, for example by down regulation of expression of the checkpoint molecules or by binding thereto and blocking normal receptor / ligand interactions. As the immune checkpoints put the brakes on the immune system response to an antigen, so an inhibitor thereof (i.e. an "immune checkpoint inhibitor") reduces this immunosuppressive effect and enhances the immune response. The immune checkpoint inhibitor for use in the invention is an inhibitor of PDL-1.

As used herein, the term "chemotherapeutic agent" is intended to broadly encompass any chemical or biological compound useful in the treatment of cancer. It includes growth inhibitory agents and other cytotoxic agents. The term "growth inhibitory agent" refers to a compound which inhibits growth of a cell, especially a cancer cell.

As used herein, the term "cancer" refers to cells undergoing abnormal proliferation. Growth of such cells typically causes the formation of a tumour. Cancerous cells may be benign, pre-malignant or malignant. Such cells may be invasive and/or have the ability to metastasize to other locations in the body. The term cancer, as used herein, includes cancerous growths, tumours, and their metastases.

The term "tumour", as used herein, refers to an abnormal mass of tissue containing cancerous cells.

As used herein, the term "metastasis" refers to the spread of malignant tumour cells from one organ or part of the body to another non-adjacent organ or part of the body. Cancer cells may break away from a primary tumour, enter the lymphatic and blood systems and circulate to other parts of the body (e.g. to normal tissues). Here they may settle and grow within the normal tissues. When tumour cells metastasize, the new tumours may be referred to as a "secondary" or metastatic cancer or tumour. The term "metastatic disease" as referred to herein relates to any disease associated with metastasis.

As used herein, the term "micrometastasis" refers to a collection of cancer cells (also known as micrometastases or "micromets") which are shed from a primary tumour and which spread to another part of the body. The term "micrometastatic disease" is used herein in respect of any disease associated with micrometastasis.

The term "circulating tumour cells" (CTCs) refers to cells that are shed into the vasculature or lymphatics from a primary tumour and are carried around the body in the blood. CTCs act as seeds for the subsequent growth of additional tumours (metastases) in other organs or parts of the body.

The term "abscopal effect" refers to a phenomenon in the treatment of metastatic cancer in which localised treatment of a tumour causes not only a reduction in the volume of the treated tumour, but also shrinkage of tumours outside of the treated area.

As used herein, "treatment" includes any therapeutic application that can benefit a human or non-human animal (e.g. a non-human mammal). Both human and veterinary treatments are within the scope of the present invention, although primarily the invention is aimed at the treatment of humans. Treatment is intended to refer to the reduction, alleviation or elimination, of a disease, condition or disorder. It includes palliative treatment, i.e. treatment intended to minimise, or partially or completely inhibit the development of the disease, condition or disorder. Where not explicitly stated, treatment also encompasses prevention. As used herein, "prevention" refers to absolute prevention, i.e. maintenance of normal levels with reference to the extent or appearance of a particular symptom of the disease, condition or disorder, or to reduction or alleviation of the extent or timing (e.g. delaying) of the onset of that symptom.

By "a pharmaceutical composition" is meant a composition in any form suitable to be used for a medical purpose.

As used herein, a "pharmaceutically effective amount" relates to an amount that will lead to the desired pharmacological and/or therapeutic effect, i.e. an amount of the agent which is effective to achieve its intended purpose. While individual subject (e.g. patient) needs may vary, determination of optimal ranges for effective amounts of the active agent(s) herein described is within the capability of one skilled in the art. Generally, the dosage regimen for treating a disease, condition or disorder with any of the agents described herein may be selected by those skilled in the art in accordance with a variety of factors including the nature of the condition and its severity.

The term "subject" refers to any individual who is the target of the administration or treatment. The subject may be, for example, a mammal. Thus the subject may be a human or non-human animal. The term "patient" refers to a subject under the treatment of a clinician. Preferably, the subject will be a human.

The microbubble-sonosensitiser complex for use in the invention comprises a microbubble attached to or otherwise associated with at least one sonosensitiser, preferably a plurality of sonosensitisers. Where the microbubble is attached to more than one sonosensitiser, these may be the same or different. Generally, however, the sonosensitisers will be identical. In the microbubble-sonosensitiser complex for use in the invention, the microbubble is attached to or otherwise associated with the sonosensitiser Rose Bengal. To the extent that such a complex is intended for use in methods of SDT, it will be ultrasound-responsive. Specifically, it is intended that the microbubble component of the complex can be ruptured by application of ultrasound, thereby releasing the sonosensitiser at the desired target site. As herein described, activation of the sonosensitiser by acoustic energy also results in the generation of reactive oxygen species, such as singlet oxygen, which are cytotoxic.

The sonosensitiser (or sonosensitisers) may be linked to the microbubble through covalent or non-covalent means, e.g. via electrostatic interaction, van der Waals forces and/or hydrogen bonding. In one embodiment the microbubble is electrostatically bound to the sonosensitiser. In another embodiment it may be covalently bound, i.e. the sonosensitiser will be attached to the microbubble by one or more covalent bonds. Preferably, however, the interaction will involve strong non-covalent bonding such as the biotin-avidin interaction.

In the case where a biotin-avidin interaction is employed to link the sonosensitiser (or sonosensitisers) to the microbubble, one component of the binding pair (e.g. the sonosensitiser) is functionalised with biotin and the other (e.g. the microbubble) with avidin. Typically, the avidin molecule will also be bound to the microbubble via a biotin-avidin interaction. For example, a microbubble may be functionalised with biotin to form a biotinylated microbubble which is then incubated with avidin. Once the avidin is bound to the bubble, this permits binding of any further biotinylated moieties, such as the sonosensitiser. The resulting linkage between the microbubble and the sonosensitiser may thus take the form a "biotin-avidin-biotin" interaction.

**In** certain embodiments, the microbubble-sonosensitiser complex may be used in combination with at least one chemotherapeutic agent (e.g. a plurality of chemotherapeutic agents) which is also linked to a microbubble. The chemotherapeutic agent (or agents) may be linked to the same microbubble as the sonosensitising agent, or alternatively it may be linked to a separate microbubble (herein a "microbubble-chemotherapeutic complex"). The "microbubble-chemotherapeutic complex" comprises a microbubble attached to or otherwise associated with at least one chemotherapeutic agent. Where any microbubble is attached to more than one chemotherapeutic agent, these may be the same or different. Generally, however, the chemotherapeutic agents attached to a particular microbubble will be identical. To the extent that the microbubble-chemotherapeutic complex is intended for use in methods of SDT, it will be ultrasound-responsive. Specifically, it is intended that the microbubble component of the complex can be ruptured by application of ultrasound, thereby releasing the chemotherapeutic agent at the desired target site.

The chemotherapeutic agent (or agents) may be linked to a microbubble through covalent or non-covalent means, e.g. via electrostatic interaction, van der Waals forces and/or hydrogen bonding. **In** one embodiment the microbubble is electrostatically bound to the chemotherapeutic agent. **In** another embodiment it may be covalently bound, i.e. the chemotherapeutic agent will be attached to the microbubble by one or more covalent bonds. Preferably, however, the interaction will involve strong non-covalent bonding such as the biotin-avidin interaction as described above. **In** the case where a biotin-avidin interaction is employed to link the chemotherapeutic agent (or agents) to the microbubble, one component of the binding pair (e.g. the chemotherapeutic agent) is functionalised with biotin and the other (e.g. the microbubble) with avidin. Typically, the avidin molecule will also be bound to the microbubble via a biotin-avidin interaction. For example, a microbubble may be functionalised with biotin to form a biotinylated microbubble which is then incubated with avidin. Once the avidin is bound to the bubble, this permits binding of any further biotinylated moieties, such as the chemotherapeutic agent. The resulting linkage between the microbubble and the chemotherapeutic agent may thus take the form a "biotin-avidin-biotin" interaction.

Where the microbubble-sonosensitiser complex for use in the invention also carries one or more chemotherapeutic agents, the sonosensitiser(s) and chemotherapeutic agent(s) may be separately linked to the microbubble, or these may be attached via a common linking group which carries both agents. This enables combined chemotherapy/SDT treatment using a single microbubble. A description of suitable linking groups is provided herein.

In certain embodiments, any of the microbubble complexes herein described may be further modified to incorporate a chemotherapeutic agent within their shell structure. For example, the microbubble may comprise a shell having incorporated therein one or more chemotherapeutic agents. Where the microbubble is linked to a chemotherapeutic agent (or agents), the chemotherapeutic agent in the shell of the microbubble may be the same or different to those which are linked to it. In one embodiment, they will be different agents.

In one embodiment the microbubble-sonosensitiser complex for use in the invention comprises a microbubble attached to or otherwise associated with at least one sonosensitising agent and at least one chemotherapeutic agent (a "first chemotherapeutic agent") wherein the microbubble comprises a shell having incorporated therein one or more additional chemotherapeutic agents (a "second chemotherapeutic agent"). Generally, the first and second chemotherapeutic agents will be different.

The choice of any of the chemotherapeutic agents for use in the invention will be dependent on various factors such as its intended use, e.g. the nature of the tumour, the patient to be treated, etc., but can readily be selected by those skilled in the art according to need.

For use in the invention, suitable classes of chemotherapeutic agents and examples within those classes include the following: antifolates (e.g. methotrexate); 5-fluoropyrimidines (e.g. 5-fluorouracil or 5-FU); cytidine analogues (e.g. gemcitabine); purine antimetabolites (e.g. mercaptopurine); alkylating agents (e.g. cyclophosphamide); non-classical alkylating agents (e.g. dacarbazine); platinum analogues (e.g. cisplatin); antitumour antibiotics (e.g. actinomycin D, bleomycin, mitomycin C); bioreductive drugs (e.g. mitomycin C, Banoxantrone (AQ4N)); anthracyclines (e.g. doxorubicin, mitoxantrone); topoisomerase I inhibitors (e.g. irinotecan); topoisomease II inhibitors (e.g. etoposide); antimicrotubule agents such as vinca alkaloids (e.g. vincristine), taxols (e.g. paclitaxel), and epothilones (e.g. ixabepiline); antioestrogens (e.g. tamoxifen); antiandrogens (e.g. biclutamide, cyproterone acetate); aromatase inhibitors (e.g. anastrazole, formestan); antiangiogenic or hypoxia targeting drugs (either naturally occuring, e.g. endostatin, or synthetic, e.g. gefitinib, lenalidomide); antivascular agents (e.g. cambretastatin); tyrosine kinase inhibitors (e.g. gefitinib, erlotinib, vandetanim, sunitinib); oncogene or signalling pathway targeting agents (e.g. tipfarnib, lonafarnib, naltrindole, rampamycin); agents targeting stress proteins (e.g. geldanamycin and analogues thereof); autophagy targeting agents (e.g. chloroquine); proteasome targeting agents (e.g. bortezomib); telomerase inhibitors (targeted oligonucleotides or nucleotides); histone deacetylase inhibitors (e.g. trichostatin A, valproic acid); DNA methyl transferase inhibitors (e.g. decitabine); alkyl sulfonates (e.g. busulfan, improsulfan and piposulfan); aziridines (e.g. benzodopa, carboquone, meturedopa, and uredopa); ethylenimines and methylamelamines (e.g. altretamine, triethylenemelamine, trietylenephosphoramide, triethylenethiophosphaoramide and trimethylolomelamine); nitrogen mustards (e.g. chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard); nitrosureas (e.g. carmustine, chlorozotocin, fotemustine, lomustine, nimustine, ranimustine); purine analogues (e.g. fludarabine, 6-mercaptopurine, thiamiprine, thioguanine); pyrimidine analogues (e.g. ancitabine, azacitidine, 6-azauridine, carmofur, cytarabine, dideoxyuridine, doxifluridine, enocitabine, floxuridine); androgens (e.g. calusterone, dromostanolone propionate, epitiostanol, mepitiostane, testolactone); anti-adrenals (e.g. aminoglutethimide, mitotane, trilostane); immune checkpoint inhibitors (e.g. the PD-1/PDL-1 interaction inhibitors BMS-1001 and BMS-1166) and other immune response modifiers (e.g. imiquimod). Pharmaceutically acceptable salts, derivatives or analogues of any of these compounds may also be used.

Examples of growth inhibitory agents for use in the invention include agents that block cell cycle progression (at a place other than S phase), such as agents that induce G1 arrest and M-phase arrest. Classical M-phase blockers include the vincas (vincristine and vinblastine); taxane family members, including paclitaxel, docetaxel, and analogues thereof; and topoisomerase inhibitors, such as irinotecan, topotecan, camptothecin, lamellarin D, doxorubicin, epirubicin, daunorubicin, etoposide, and bleomycin. Those agents that arrest G1 include, for example, DNA alkylating agents, such as tamoxifen, prednisone, dacarbazine, mechlorethamine, cisplatin, methotrexate, 5-FU, and ara-C.

In one embodiment the chemotherapeutic agent may be an anti-metabolite. Anti-metabolites which are particularly suitable for use in the invention include the 5-fluoropyrimidines, and cytidine analogues. Examples of anti-metabolites which may be used in the treatment of pancreatic cancer are 5-fluorouracil (5-FU) and gemcitabine.

In one embodiment the chemotherapeutic agent is a growth inhibitory agent. Those which are particularly suitable for use in the invention include the anthracycline topoisomerase inhibitors, e.g. doxorubicin.

For incorporation within the shell structure of the microbubble, any of the chemotherapeutic agents herein described may be chosen. Preferably, the chemotherapeutic agent should be one capable of spontaneously embedding within the hydrophobic lipid chains of the microbubble lipids. This may involve direct hydrophobic interaction in cases where the chemotherapeutic is hydrophobic. In one embodiment, the chemotherapeutic agent for incorporation within the shell of the microbubble may therefore be hydrophobic. Hydrophobic agents may be considered to be those having a LogP value greater than about 2. Alternatively, non-polar chemotherapeutic agents may be suitably modified (e.g. functionalised) by the introduction or one or more non-polar functional groups which enable them to spontaneously embed within the shell (e.g. the lipid shell) of the microbubble.

In one embodiment, the chemotherapeutic agent to be incorporated within the shell of the microbubble may be an anti-microtubule agent. Examples of such agents include, in particular, taxols such as paclitaxel. Paclitaxel (or "PTX") is hydrophobic. In another embodiment, an immune checkpoint inhibitor (e.g. BMS-1001 or BMS-1166) may be included within the shell of the microbubble.

In one embodiment, the microbubble-sonosensitiser complex for use in the invention is one in which the sonosensitiser is Rose Bengal and has a taxol (e.g. paclitaxel) embedded within the shell of the microbubble. In another embodiment, the microbubble-sonosensitiser complex for use in the invention has a taxol (e.g. paclitaxel) embedded within the shell of the microbubble, and is linked to a chemotherapeutic agent which is an anti-metabolite (e.g. gemcitabine) or a topoisomerase inhibitor (e.g. doxorubicin).

Microbubbles are well known in the art, for example as ultrasound contrast agents. Their composition and methods for their preparation are thus well known to those skilled in the art. Examples of procedures for the preparation of microbubbles are described in, for example, Christiansen et al., Ultrasound Med. Biol., 29: 1759-1767, 2003; Farook et al., J. R. Soc. Interface, 6: 271-277, 2009; and Stride & Edirisinghe, Med. Biol. Eng. Comput., 47: 883-892, 2009.

Microbubbles comprise a shell which surrounds an internal void comprising a gas. Generally, these are approximately spherical in shape, although the shape of the microbubble is not essential in carrying out the invention and is therefore not to be considered limiting. The size of the microbubble should be such as to permit its passage through systemic circulation (e.g. the pulmonary system) following administration, e.g. by intravenous injection. Microbubbles typically have a diameter of less than about 200 µm, preferably in the range from about 0.1 to about 100 µm, e.g. from about 0.5 to about 100 µm. Particularly suitable for use in the invention are microbubbles having a diameter of less than about 10 µm, more preferably 1 to 8 µm, particularly preferably up to 5 µm, e.g. 1 to 3 µm or about 2 µm. The shell of the microbubble will vary in thickness and will typically range from about 5 to about 200 nm, e.g. from about 10 to about 200 nm. The precise thickness is not essential provided that the shell performs the desired function of retaining the gas core.

Materials which may be used to form the microbubbles should be biocompatible and suitable materials are well known in the art. Typically, the shell of the microbubble will comprise a surfactant or a polymer. Surfactants which may be used include any material which is capable of forming and maintaining a microbubble by forming a layer at the interface between the gas within the core and an external medium, e.g. an aqueous solution which contains the microbubble. A surfactant or combination of surfactants may be used. Those which are suitable include lipids, in particular phospholipids. Lipids which may be used include lecithins (i.e. phosphatidylcholines), e.g. natural lecithins such as egg yolk lecithin or soya bean lecithin and synthetic lecithins such as dimyristoylphosphatidylcholine, dipalmitoylphosphatidylcholine or distearoylphosphatidylcholine; phosphatidic acids; phosphatidylethanolamines; phosphatidylserines; phosphatidylglycerols; phosphatidylinositols; and mixtures thereof. In one embodiment, lipids such as 1,2-dibehenoyl-sn-glycero-3-phosphocholine (DBPC) and/or 1,2-distearoyl-sn-glycero-3-phosphoethanolamine (DSPE) may be used to form the shell of the microbubbles. Combinations of DBPC and DSPE are particularly suitable.

Suitable lipids and combinations of lipids may be selected based on their ability to enhance the stability of the microbubbles with regard to oxygen retention. Suitable for use in this regard is 1,2-dibehenoyl-sn-glycero-3-phosphocholine (DBPC). In one embodiment, a combination of lipids may be used in which DBPC is present in an amount of at least 70, preferably at least 80, more preferably at least 80 mol.% (based on the total amount of lipid).

Polymer materials which are suitable for use in forming the shell of the microbubble include proteins, in particular albumin, particularly human serum albumin. Other biocompatible polymers which may be used include poly(vinyl alcohol) (PVA), poly(D,L-lactide-co-glycolide) (PLGA), cyanoacrylate, poloxamers (Pluronics), chitosan and chitosan derivatives, or combinations thereof.

The microbubble shells may comprise single or multiple layers of the same or different materials. Multiple layers may, for example, be formed in cases where the basic shell material (e.g. a lipid) bears one or more polymers or polysaccharides. Examples of such polymers include polyethylene glycol (PEG) and polyvinylpyrrolidone. The microbubble shell may also be coated with polymers, such as poly-L-lysine and PLGA, and/or polysaccharides, such as alginate, dextran, diethylamino-ethyl-dextran hydrochloride (DEAE) or chitosan. Methods for attaching these coating materials may involve electrostatic or covalent interactions. Different coating materials (polymers, polysaccharides, proteins, etc.) may be used in order to improve the properties of the microbubble, for example by increasing the rigidity, stability in circulation and/or tissue permeation capability of the microbubble-based reagents, by manipulating the net surface charge of the microbubble and, perhaps most importantly, by increasing its payload capacity. Lipids forming either a monolayer, bilayer or multilamellar structure may be used to form the microbubbles for use in the invention. Examples of these include unilamellar or multilammellar liposomes and micelles.

Any of the microbubble shells herein described may comprise further components which aid in accumulation of the microbubbles at the target site. For example, these may be functionalised such that these incorporate or have bound thereto a ligand or targeting agent which is able to bind to a target cell or tissue. Examples of suitable targeting agents include antibodies and antibody fragments, cell adhesion molecules and their receptors, cytokines, growth factors and receptor ligands. Such agents can be attached to the microbubbles using methods known in the art, e.g. by covalent coupling, the use of molecular spacers (e.g. PEG) and/or the avidin-biotin complex method. For example, the incorporation of a lipid-PEG-biotin conjugate in lipid-based microbubbles followed by the addition of avidin enables functionalisation of the microbubble surface with a biotinylated targeting ligand. Herceptin is an example of an antibody which may be conjugated to the microbubble shell for targeting purposes.

The microbubble shell may further comprise components which aid in its attachment to one or more linking groups as herein described. **In** one embodiment, the microbubble shell may be covalently coupled to biotin or a biotin residue via a molecular spacer, such as PEG (e.g. PEG-2000). This enables functionalisation of the surface of the microbubble with avidin which may then be conjugated to a biotinylated linking group. Incorporation of a lipid-spacer-biotin conjugate (e.g. a lipid-PEG-biotin conjugate) in the shell of the microbubble may be achieved by appropriate functionalisation of one or more lipids prior to formation of the microbubble.

The gas within the core of the microbubble should be biocompatible. The term "gas" encompasses not only substances which are gaseous at ambient temperature and pressure, but also those which are in liquid form under these conditions. Where the "gas" is liquid at ambient temperature this will generally undergo a phase change to a gas or vapour at a temperature of 38°C or above. For any gas which is a liquid at ambient temperature, it is generally preferred that this will undergo a phase change to a gas at a temperature between about 38 and 45°C, preferably slightly above body temperature. For example, it may undergo a phase change when subjected to a stimulus, such as ultrasound, which causes a local increase in temperature. Any reference herein to "gas" should thus be considered to encompass not only gases and liquids, but also liquid vapours and any combination thereof, e.g. a mixture of a liquid vapour in a gas.

Gases which are suitable for incorporation within the microbubbles for use according to the invention include air, nitrogen, oxygen, carbon dioxide, hydrogen; inert gases such as helium, argon, xenon or krypton; sulphur fluorides such as sulphur hexafluoride, disulphur decafluoride; low molecular weight hydrocarbons such as alkanes (e.g. methane, ethane, propane, butane), cycloalkanes (e.g. cyclopropane, cyclobutane, cyclopentane), alkenes (e.g. ethylene, propene); and alkynes (e.g. acetylene or propyne); ethers; esters; halogenated low molecular weight hydrocarbons; and mixtures thereof.

Examples of suitable halogenated hydrocarbons are those which contain one or more fluorine atoms and include, for example, bromochlorodifluoromethane, chlorodifluoromethane, dichlorodifluoromethane, bromotrifluoromethane, chlorotrifluoromethane, chloropentafluoroethane, dichlorotetrafluoroethane, chlorotrifluoroethylene, fluoroethylene, ethyl fluoride, 1,1-difluoroethane and perfluorocarbons. Examples of suitable fluorocarbon compounds include perfluorocarbons. Perfluorocarbons include perfluoroalkanes such as perfluoromethane, perfluoroethane, perfluoropropanes, perfluorobutanes, perfluoropentanes, perfluorohexanes and perfluoroheptanes; perfluoroalkenes such as perfluoropropene, perfluorobutenes; and perfluorocycloalkanes such as perfluorocyclobutane. Microbubbles containing perfluorinated gases, in particular, perfluorocarbons such as perfluoropropanes, perfluorobutanes, perfluoropentanes and perfluorohexanes are suitable for use in the invention due to their stability in the bloodstream.

Microbubbles containing a perfluorocarbon, particularly a perfluoroalkane, and a shell comprising a phospholipid may be used in the invention and are described in, for example, Nomikou & McHale, Cancer Lett., 296: 133-143, 2010. One example of such a microbubble is Sonidel SDM202 (available from Sonidel Ltd.). The perfluorocarbon may either be present as a gas or in liquid form. Those containing a liquid core may be prepared from nanoemulsions which may subsequently be converted to a gas microbubble upon exposure to ultrasound, e.g. as described in Rapoport et al., Bubble Sci. Eng. Technol. 1: 31-39, 2009.

In one embodiment, the microbubbles for use in the invention may carry oxygen (e.g. oxygen gas). As oxygen is a key substrate for SDT and many cancers are hypoxic, filling the core of the bubble with oxygen gas enhances the sonodynamic effect and the amount of singlet oxygen produced. Since PDAC possesses a pronounced hypoxic tumour environment which can also suppress the immune system, the use of oxygen-loaded MBs as herein described which simultaneously deliver oxygen and their attached payloads to pancreatic tumours is particularly beneficial both from a therapeutic and immunological perspective.

The sonosensitiser for use in the invention is Rose Bengal.

Methods for the formation of microbubbles are known in the art. Such methods include the formation of a suspension of the gas in an aqueous medium in the presence of the selected shell material. Techniques used to form the microbubble include sonication, high speed mixing (mechanical agitation), coaxial electrohydrodynamic atomisation and microfluidic processing using a T-junction (see e.g. Stride & Edirisinghe, Med. Biol. Eng. Comput., 47: 883-892, 2009). Sonication is widely used and generally preferred. This technique may be carried out using an ultrasound transmitting probe. More particularly, an aqueous suspension of the microbubble shell components is sonicated in the presence of the relevant microbubble component gas, e.g. oxygen.

Other methods which may be used to form the microbubbles include vaporisation of a nanodroplet core in a nanoemulsion (see e.g. Rapoport *et al., supra).* The core of such nanodroplets will typically be formed by an organic perfluorocompound which is encased by a lipid shell or a biodegradable amphiphilic block copolymer such as poly(ethylene oxide)-co-poly(L-lactide) or poly(ethylene oxide)-co-caprolactone. Alternatively, nanoemulsions may be prepared by extrusion through sizing membranes, for example using albumin as the shell material. The droplet-to-bubble transition may be induced by physical and/or mechanical means which include heat, ultrasound and injection through a fine-gauge needle. Such microbubbles may be formed at the point of administration to the patient (e.g. during the step of administration using a fine-gauge needle) or *in vivo* at the desired target cells or tissues (e.g. by subjecting the nanoemulsion to ultrasound).

Administration of a nanodroplet which is capable of forming the desired microbubble complex as herein defined, either during the step of administration to the patient or post-administration (i.e. *in vivo*), is within the scope of the present invention. Where it is desired that the resulting microbubble contains oxygen gas, this may be provided in dissolved form in a liquid perfluorocarbon core of a phase-shift nanoemulsion.

The microbubble complexes herein described may be prepared using methods and procedures known in the art. Methods which may be used for covalently attaching the sonosensitising agent and/or the chemotherapeutic agent to a microbubble include known chemical coupling techniques. The exact method used will be dependent on the exact nature of the microbubble, the sonosensitising agent and the chemotherapeutic agent, specifically the nature of any pendant functional groups. If necessary, one or both components which are to be linked may be functionalised, e.g. to include reactive functional groups which may be used to couple the molecules. Suitable reactive groups include acid, hydroxy, carbonyl, acid halide, thiol and/or primary amine. Methods for the introduction of such functional groups are well known in the art.

Examples of methods which may be used to covalently bind a microbubble to one or more sonosensitising agents and/or chemotherapeutic agents include, but are not limited to, the following: a) Carbodiimide based coupling methods. These may be used to couple microbubbles containing either an amine or carboxylic acid functionality to a moiety having either a carboxylic acid or amine functionality. Such methods result in the formation of ester or amide bonds; b) "CLICK" reaction (i.e. 1,3-dipolar cycloaddition reaction). This may be used to react azide or acetylene functionalised microbubbles with a moiety having either acetylene or azide functionality; c) Schiff base formation (i.e. imine bond formation). This reaction may be used to bond aldehyde or amine functionalised microbubbles to a moiety containing amine or aldehyde functionality; and d) Michael addition reactions.

Linkage of the microbubble to one or more sonosensitisers and/or chemotherapeutic agents, or to one or more linking groups as herein described, via the biotin-avidin linkage may be carried out by methods known to those skilled in the art. In such methods, both moieties will typically be biotinylated and avidin then used to form the linkage between the two. An example of a method to produce a microbubble-sonosensitiser complex in which the microbubble is bound to a tripodal linking group via a biotin-avidin-biotin interaction is provided in Scheme 3 in Example 4.

As an alternative to coupling of any of the sonosensitiser, chemotherapeutic agent, or linking groups herein described to a pre-formed microbubble, these moieties may alternatively be linked to a lipid (e.g. using any of the methods described above) and that lipid may subsequently be incorporated into the lipid shell of the microbubble during its preparation.

Charged sonosensitisers and/or chemotherapeutic agents may be electrostatically linked to a charged microbubble. For example, an anionic bubble may be linked to a cationic sonosensitiser or cationic chemotherapeutic agent and *vice versa.*

Examples of methods for the preparation of a microbubble-sonosensitiser complex are disclosed in WO 2012/143739. Examples of methods for the preparation of microbubbles carrying sonosensitisers and/or chemotherapeutic agents are disclosed in WO 2017/089800.

In the case where a chemotherapeutic agent (e.g. paclitaxel, PTX) is incorporated within the shell of the microbubble this may, for example, be dissolved in an organic solvent and added to a solution containing the lipids prior to formation of the microbubble. Evaporation of the solvent provides a dried lipid film incorporating the chemotherapeutic agent (e.g. PTX) which may be reconstituted and sonicated to provide the loaded microbubble. The lipid-chemotherapeutic agent film (e.g. lipid-PTX film) may be reconstituted in a suitable solvent, heated above the lipid transition temperature and gently sonicated to ensure full incorporation of the chemotherapeutic agent into the lipid chains. The solution may then be sparged with a suitable gas (e.g. perfluorobutane, PFB) while sonicating to prepare the final microbubble suspension.

Where the shell comprises polymer materials, such as albumin, a chemotherapeutic agent (e.g. paclitaxel) may be incorporated (e.g. embedded) within the shell of the microbubble using a double emulsion (e.g. water-in-oil-in-water) method. Using this method, the chemotherapeutic agent may be dissolved in the oil phase of the emulsion along with the polymer. Following removal of a solvent from the emulsion, the oil phase becomes a polymer shell having the chemotherapeutic agent embedded therein.

In certain embodiments, the microbubble-sonosensitiser complex for use in the invention may simultaneously deliver both the sonosensitiser and a chemotherapeutic agent. In these embodiments, the microbubble is used as a carrier for both agents. Where both agents are linked to a single microbubble these may be attached via separate linking groups, such as described in WO 2017/089800 for example. Alternatively, a ligand (referred to herein as a "linking group") may be used to attach both the sonosensitiser and the chemotherapeutic agent to the surface of a single microbubble (e.g. via the "biotin-avidin" interaction). This enables combined chemotherapy/SDT treatment using a single microbubble. By attaching both agents to the same microbubble rather than to separate microbubbles, an increase in drug loading can be achieved. The ligand may also be modified to carry two or more chemotherapeutic agents and/or two or more sonosensitisers to further enhance drug loading.

The microbubble-sonosensitiser complexes for use according to the invention may thus comprise a microbubble attached to or otherwise associated with at least one sonosensitising agent and at least one chemotherapeutic agent. In one embodiment, these agents may be attached to or otherwise associated with the microbubble via one or more linking groups which each carry at least one sonosensitising agent and at least one chemotherapeutic agent.

**In** one embodiment the microbubble-sonosensitiser complex for use according to the invention comprises a microbubble attached to or otherwise associated with a plurality of sonosensitising agents and a plurality of chemotherapeutic agents. Where the microbubble is bound to more than one sonosensitising agent, these may be the same or different, and may be carried by a single linking group or two or more linking groups. Generally, the sonosensitising agents bound to a particular microbubble will be identical. **In** the microbubble-sonosensitiser complex for use in the invention, the microbubble is attached to or otherwise associated with the sonosensitising agent Rose Bengal. Where the microbubble is bound to more than one chemotherapeutic agent, these may be the same or different, and may be carried by a single linking group or two or more linking groups. Generally, the chemotherapeutic agents attached to a particular microbubble via a linking group (or groups) as herein described will be identical.

The chemotherapeutic agent(s) and sonosensitising agent(s) may be linked to the microbubble via one or more linking groups. Each linking group may be bound to or otherwise associated with the microbubble and the chemotherapeutic agent(s) and sonosensitising agent(s) through covalent or non-covalent means, e.g. via electrostatic interaction, hydrophobic interactions, van der Waals forces, hydrogen bonding, or any combination thereof. **In** one embodiment the interaction between the linking group(s) and the microbubble may involve strong non-covalent bonding such as the biotin-avidin interaction. **In** this embodiment one component of the binding pair (e.g. the linking group) is functionalised with biotin and the other (e.g. the microbubble) with avidin. Since avidin contains multiple binding sites for biotin, this will typically also be bound to the microbubble via a biotin-avidin interaction. For example, a microbubble may be functionalised with biotin to form a biotinylated microbubble which is then incubated with avidin. Once the avidin is bound to the microbubble, this permits binding of any further biotinylated moieties, such as a linking group which incorporates biotin (or a biotin residue). The resulting linkage between the microbubble and the linking group may thus take the form of a "biotin-avidin-biotin" interaction.

In one embodiment, the chemotherapeutic agent(s) and/or sonosensitising agent(s) are covalently bound to the linking group(s), i.e. the chemotherapeutic agent(s) and/or sonosensitising agent(s) are attached to the linking group(s) via one or more covalent bonds.

As will be understood, the precise nature of the linking group(s) is not critical provided these are capable of linking at least one chemotherapeutic agent and at least one sonosensitising agent to the microbubble (or to a suitably 'functionalised' microbubble as herein described, e.g. a microbubble which carries one or more biotin-avidin functionalities). As will be appreciated, any linking group should be biocompatible.

Any of the microbubbles herein described may be bound to or otherwise associated with a plurality of linking groups in order to further increase the loading of sonosensitising and chemotherapeutic agents. In this embodiment, the linking groups need not be identical to one another although generally they will be the same.

Suitable linking groups may readily be selected by those skilled in the art. Typically each linking group will comprise an organic group comprising a chain of up to about 200 atoms, e.g. up to about 100 atoms, between its points of attachment to the microbubble (or 'functionalised' microbubble) and to the chemotherapeutic agent and sonosensitising agent. The organic chain may comprise aliphatic, alicyclic, or aromatic groups, or any combination thereof. In one embodiment, it may comprise aliphatic, alicyclic and aromatic groups. In another embodiment, it may comprise aliphatic and alicyclic groups. Suitable linking groups may have a molecular weight of up to about 3,000 Da, e.g. up to about 1,500 Da.

Suitable linking groups may be linear or branched. In one embodiment the linking group may be branched. Various degrees of branching may be provided and can be selected depending on the number of agents to be carried by the linking group. For example, the linking group may comprise up to six branches, e.g. one, two, three or four branches, which enable its attachment to the microbubble (or 'functionalised' microbubble), and to the chemotherapeutic agent(s) and sonosensitising agent(s). Attachment of the linking group to the microbubble and to the chemotherapeutic and sonosensitising agents will generally be via terminal groups of the branched structure.

In one embodiment the linking group may comprise three branches, i.e. it is "tri-podal". In this embodiment, a first branch of the linking group will be capable of binding to the microbubble (e.g. via a non-covalent interaction such as "avidin-biotin"), a second branch will be capable of linking to the chemotherapeutic agent (e.g. covalently), and a third branch will be capable of linking to the sonosensitising agent (e.g. covalently).

Suitable linking groups may comprise a straight-chained or branched (preferably branched) C₃₀₋₅₀ alkylene chain (preferably a C₃₀₋₄₀ alkylene chain) optionally substituted by one or more groups selected from C₁₋₃ alkyl, -O(C₁₋₃)alkyl, and -OR' (where R' is H or C₁₋₆ alkyl, preferably C₁₋₃ alkyl, e.g. methyl); and in which one or more (preferably up to 10, e.g. from 4 to 9, or from 6 to 8) -CH₂- groups of the alkylene chain may be replaced by a group independently selected from -O-, -CO-, -C(O)O-, -NR"- and -NR"CO- (where each R" is independently H or C₁₋₆ alkyl, preferably C₁₋₃ alkyl, e.g. methyl). In one embodiment the linking group may also be substituted (e.g. terminally substituted) by biotin or a biotin residue as herein described.

In one embodiment, the linking group may comprise one or more amino acids. For example, it may comprise a peptide, a peptide residue or fragment.

Tri-podal linking groups suitable for use in the invention include those in which the branches are linked to a central N or C atom. Those having a central nitrogen atom are preferred for use in the invention. Such linking groups include those having the following structure: 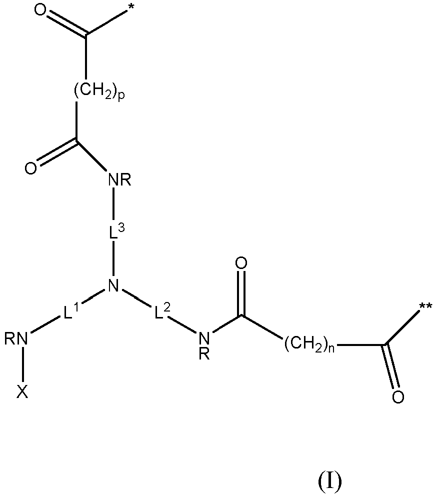 wherein L¹, L² and L³ are each independently -(CH₂)_{q}- in which q is an integer from 1 to 4, preferably 2; each R is independently either H or C₁₋₆ alkyl (preferably C₁₋₃ alkyl, e.g. CH₃), preferably H; n is an integer from 2 to 10, preferably 4 to 8, more preferably 5 to 7, e.g. 6; p is an integer from 2 to 10, preferably 4 to 8, more preferably 5 to 7, e.g. 6; X is a functional group capable of binding to a microbubble or to a 'functionalised' microbubble as herein described; * denotes the point of attachment of the linking group to a sonosensitising agent, a 'functionalised' sonosensitising agent, or a residue of a sonosensitising agent as herein described; and ** denotes the point of attachment of the linking group to a chemotherapeutic agent, a 'functionalised' chemotherapeutic agent, or a residue of a chemotherapeutic agent as herein described.

As will be understood, linkage of the various components to form the microbubble complexes according to the invention may, in some cases, require that one or more of the components are suitably "functionalised", for example by incorporation of one or more reactive groups which enable their linkage or association with one another (e.g. by the formation of a covalent bond, or any other type of bonding herein described). Any reference herein to a "functionalised" component of the complexes should be construed accordingly. For example, a "functionalised" microbubble may carry a 'biotin-avidin' functional group in order that it may bind to a biotinylated linking group. "Functionalised" sonosensitising agents and "functionalised" chemotherapeutic agents may, for example, carry one or more reactive groups (such as amine, e.g. primary amine, carboxyl, hydroxyl, acid, acid halide, thiol, carbonyl, etc.) which enable their linkage to the chosen linking group. Any suitable functional groups may be used and these may readily be selected by those skilled in the art depending on the nature of the components to be linked to one another.

"Functionalisation" of any component (e.g. the sonosensitising agent or the chemotherapeutic agent) will typically involve reaction with one or more compounds which are capable of providing the desired "functionalised" component. Suitable compounds may readily be determined by any skilled chemist and may include, for example, moieties containing an amine or carboxylic acid group. Following reaction with the agent these may, for example, provide a terminal amine or carboxylic acid functionality which is capable of reaction with the chosen linking group. Examples of compounds which may be used for functionalisation of a sonosensisting agent or a chemotherapeutic agent are illustrated herein in Schemes 3 and 5. In Scheme 3, Br-CH₂-CH₂-NH₂ is used to functionalise the sonosensitising agent Rose Bengal to produce "Rose Bengal-amine". In Scheme 6, Rose Bengal is reacted with 8-bromooctanoic acid to produce "Rose Bengal-octanoic acid", and the chemotherapeutic agent gemcitabine is reacted with HO-(CH₂)₁₁-CO₂H and 4-nitrophenyl chloroformate to produce a carboxylic acid functionalised gemcitabine.

Similarly, it will be understood that following linkage of the various components (e.g. via a chemical reaction) to form the microbubble complexes for use according to the invention, some or all of the components may no longer retain their original structure but may "lose" one or more terminal groups or atoms (e.g. a H atom) as a result of the reaction involved in their linkage or association with one another (e.g. by the formation of a covalent bond, or any other type of bonding herein described). These components may be considered a "residue" of the original component and any reference to a "residue" of a component of the complexes should be construed accordingly. Schemes 3 and 6 are provided herein as examples of methods which may be used in the preparation of a tri-podal linking group incorporating biotin. In these examples, the terminal carboxyl group of biotin is used to link it to the tri-podal linking group, typically via an amine or ester bond. In the final structure the biotin is present as a "residue" of biotin.

In formula (I), L¹, L² and L³ may be identical. In one embodiment, L¹, L² and L³ are each -(CH₂)₂-. In formula (I), each R may be identical. In one embodiment, each R is H. In formula (I), n and p may be identical. In one embodiment, n and p are both an integer from 4 to 8, e.g. 6. In one embodiment of formula (I), X is biotin or a biotin residue which is capable of binding to an avidin-functionalised microbubble.

An example of a tri-podal linking group within general formula (I) for use in the invention is as follows: 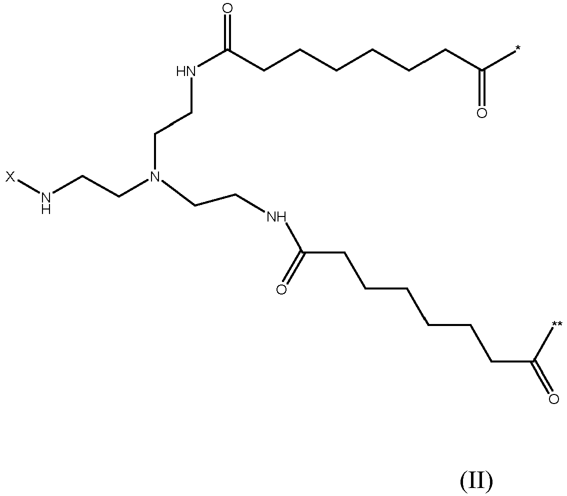 wherein X is a functional group capable of binding to a microbubble or to a 'functionalised' microbubble as herein described; * denotes the point of attachment of the linking group to a sonosensitising agent, a 'functionalised' sonosensitising agent, or a residue of a sonosensitising agent as herein described; and ** denotes the point of attachment of the linking group to a chemotherapeutic agent, a 'functionalised' chemotherapeutic agent, or a residue of a chemotherapeutic agent as herein described. In one embodiment of formula (II), X is biotin or a biotin residue which is capable of binding to an avidin-functionalised microbubble.

Other tri-podal linking groups which may be used in the invention are those having the following general structure: 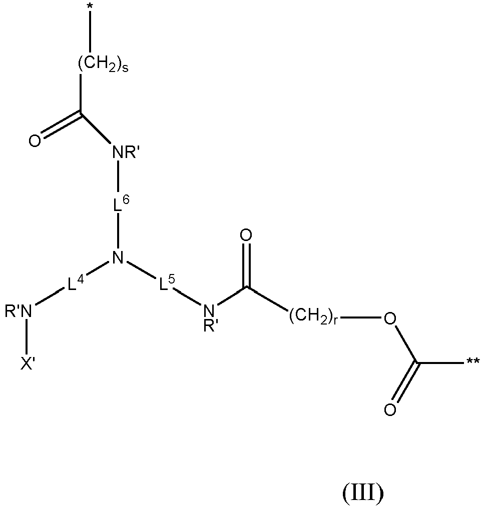 wherein L⁴, L⁵ and L⁶ are each independently -(CH₂)ₜ- in which t is an integer from 1 to 4, preferably 2; each R' is independently either H or C₁₋₆ alkyl (preferably C₁₋₃ alkyl, e.g. CH₃), preferably H; r is an integer from 2 to 10, preferably 4 to 8, more preferably 5 to 7, e.g. 6; s is an integer from 2 to 10, preferably 4 to 8, more preferably 5 to 7, e.g. 6 or 7; X' is a functional group capable of binding to a microbubble or to a 'functionalised' microbubble as herein described; * denotes the point of attachment of the linking group to a sonosensitising agent, a 'functionalised' sonosensitising agent, or a residue of a sonosensitising agent as herein described; and ** denotes the point of attachment of the linking group to a chemotherapeutic agent, a 'functionalised' chemotherapeutic agent, or a residue of a chemotherapeutic agent as herein described.

In an alternative embodiment of formula (III): * denotes the point of attachment of the linking group to a chemotherapeutic agent, a 'functionalised' chemotherapeutic agent, or a residue of a chemotherapeutic agent as herein described; and **denotes the point of attachment of the linking group to a sonosensitising agent, a 'functionalised' sonosensitising agent, or a residue of a sonosensitising agent as herein described.

In formula (III), L⁴, L⁵ and L⁶ may be identical. In one embodiment, L⁴, L⁵ and L⁶ are each -(CH₂)₂-. In formula (III), each R' may be identical. In one embodiment, each R' is H. In formula (III), r and s may be identical or different. In one embodiment, r and s are both an integer from 4 to 8, e.g. 6 or 7. In one embodiment r is 6 and s is 7. In one embodiment of formula (III), X' is biotin or a biotin residue which is capable of binding to an avidin-functionalised microbubble.

An example of a tri-podal linking group according to formula (III) which may be used in the invention is as follows: 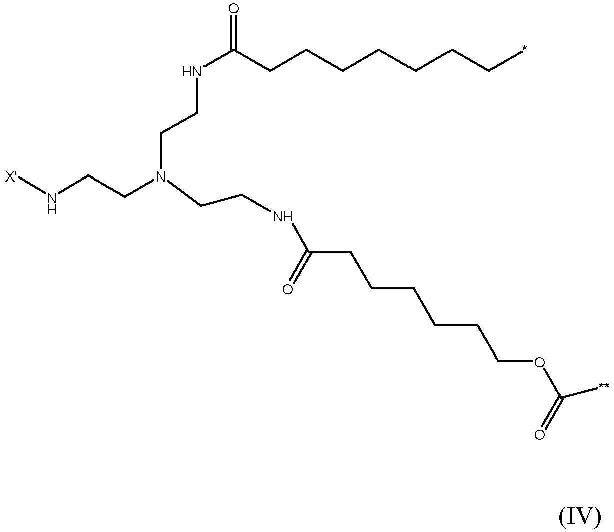 wherein X' is a functional group capable of binding to a microbubble or to a 'functionalised' microbubble as herein described; * denotes the point of attachment of the linking group to a sonosensitising agent, a 'functionalised' sonosensitising agent, or a residue of a sonosensitising agent as herein described; and ** denotes the point of attachment of the linking group to a chemotherapeutic agent, a 'functionalised' chemotherapeutic agent, or a residue of a chemotherapeutic agent as herein described.

In one embodiment of formula (IV), X' is biotin or a biotin residue which is capable of binding to an avidin-functionalised microbubble.

Other tri-podal linking groups which may be used in the invention are those having the following general structure: 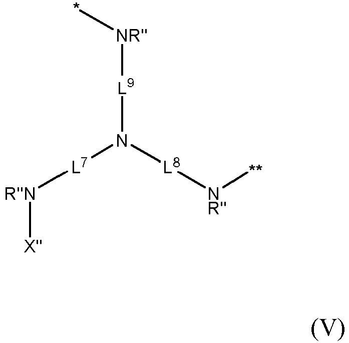 wherein L⁷, L⁸ and L⁹ are each independently -(CH₂)ᵤ- in which u is an integer from 1 to 4, preferably 2; each R'' is independently either H or C₁₋₆ alkyl (preferably C₁₋₃ alkyl, e.g. CH₃), preferably H; X'' is a functional group capable of binding to a microbubble or to a 'functionalised' microbubble as herein described; * denotes the point of attachment of the linking group to a sonosensitising agent, a 'functionalised' sonosensitising agent, or a residue of a sonosensitising agent as herein described; and ** denotes the point of attachment of the linking group to a chemotherapeutic agent, a 'functionalised' chemotherapeutic agent, or a residue of a chemotherapeutic agent as herein described.

In formula (V), L⁷, L⁸ and L⁹ may be identical. In one embodiment, L⁷, L⁸ and L⁹ are each -(CH₂)₂-. In formula (V), each R'' may be identical. In one embodiment, each R'' is H. In one embodiment of formula (V), X'' is biotin or a biotin residue which is capable of binding to an avidin-functionalised microbubble.

An example of a tri-podal linking group according to formula (V) which may be used in the invention is as follows: 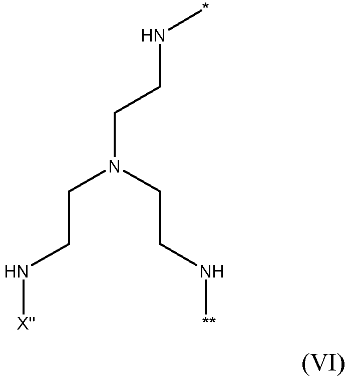 wherein X" is a functional group capable of binding to a microbubble or to a 'functionalised' microbubble as herein described; * denotes the point of attachment of the linking group to a sonosensitising agent, a 'functionalised' sonosensitising agent, or a residue of a sonosensitising agent as herein described; and ** denotes the point of attachment of the linking group to a chemotherapeutic agent, a 'functionalised' chemotherapeutic agent, or a residue of a chemotherapeutic agent as herein described.

The microbubble-sonosensitiser complexes herein described are used in a method of combination therapy in which they are administered to a subject (e.g. to a patient) in combination with a separate immune checkpoint inhibitor. The immune checkpoint inhibitor for use in the combination therapy according to the invention is an inhibitor of PDL-1.

The invention thus provides, in one aspect, a microbubble-sonosensitiser complex for use in the treatment of metastatic disease via sonodynamic therapy, wherein said sonodynamic therapy comprises simultaneous, separate or sequential use of an immune checkpoint inhibitor; and further wherein said sonosensitiser is Rose Bengal and said immune checkpoint inhibitor is an inhibitor of PDL-1.

Also disclosed herein, but not claimed, is a method of sonodynamic therapy which comprises at least the following steps:
(a) administering a microbubble-sonosensitiser complex as herein described to affected cells or tissues of a subject in need thereof (e.g. a patient) and subjecting said cells or tissues to ultrasound irradiation whereby to activate said complex; and
(b) simultaneously, separately or sequentially administering to said subject (e.g. said patient) a pharmaceutically effective amount of an immune checkpoint inhibitor which is an inhibitor of PDL-1.

When used in combination therapy, the microbubble-sonosensitiser complex and immune checkpoint inhibitor may be administered to the subject separately, simultaneously or sequentially. Where they are administered sequentially, they may be administered in either order. In one embodiment, the immune checkpoint inhibitor is administered prior to the microbubble-sonosensitiser complex. For example, it may be administered up to several hours or even several days prior to administration of the microbubble-sonosensitiser complex. In particular, the immune checkpoint inhibitor may be administered from 1 hour to 5 days, e.g. from 2 hours to 3 days before the microbubble-sonosensitiser complex is administered and SDT is carried out.

In an embodiment of the invention, the microbubble-sonosensitiser complex and immune checkpoint inhibitor will be administered separately from one another, preferably sequentially.

A product comprising a microbubble-sonosensitiser complex as herein described and an immune checkpoint inhibitor which is an inhibitor of PDL-1 for simultaneous, separate or sequential use in the treatment of metastatic disease via sonodynamic therapy also forms part of the invention.

Also disclosed herein, but not claimed, is a kit (or pharmaceutical pack) comprising the following components: (i) a microbubble-sonosensitiser complex as herein described; and separately (ii) an immune checkpoint inhibitor which is an inhibitor of PDL-1; optionally together with (iii) instructions for the use of said components in a method of sonodynamic therapy.

For simultaneous administration, the microbubble-sonosensitiser complex and immune checkpoint inhibitor may be provided together in a single formulation. In another aspect, the invention thus provides a pharmaceutical composition comprising a microbubble-sonosensitiser complex as herein described and an immune checkpoint inhibitor which is an inhibitor of PDL-1, together with at least one pharmaceutical carrier or excipient.

The immune checkpoint inhibitor for use in the invention is a PDL-1 inhibitor. Examples of such drugs are known and used in the art and any may be suitable for use in the invention.

Examples of PDL-1 inhibitors which may be used in the invention include, but are not limited to, atezolizumab (MPDL3280A), avelumab, and duravlumab.

When used in combination therapy with the immune checkpoint inhibitor, the microbubble-sonosensitiser complexes herein described are suitable for the treatment of cancerous growths or tumours, and their metastases.

The following tumours and associated metastatic conditions may be treated: pancreatic cancer, breast cancer, prostate cancer, glioma, non-small cell lung carcinoma, head and neck cancers, cancers of the urinary tract, kidney or bladder, advanced melanoma, oesophageal cancer, colon cancer, hepatic cancer, and lymphoma. Metastatic disease arising from any of these tumours may be treated using any of the methods herein described. The treatment of metastatic pancreatic cancer forms a preferred aspect of the invention.

The methods herein described find particular use in the treatment of patients who have undergone conventional immunotherapy (e.g. treatment with an immune checkpoint inhibitor) either alone, or in combination with other chemotherapeutic, radiotherapeutic and/or ablative procedures, without success. Ablative cancer treatments involve the use of heat or cold to destroy, or ablate tumours without the need for invasive surgery. Examples of ablative therapy include thermal ablation, radiofrequency ablation, microwave ablation, and high-intensity focused ultrasound ablation.

For use in any of the methods herein described, the microbubble complexes will generally be provided in a pharmaceutical composition together with at least one pharmaceutically acceptable carrier or excipient. Such pharmaceutical compositions may be formulated using techniques well known in the art. The route of administration will depend on the intended use. Typically, these will be administered systemically and may thus be provided in a form adapted for parenteral administration, e.g. by intradermal, subcutaneous, intraperitoneal or intravenous injection. Suitable pharmaceutical forms include suspensions and solutions which contain the active microbubble complexes together with one or more inert carriers or excipients. Suitable carriers include saline, sterile water, phosphate buffered saline and mixtures thereof. The compositions may additionally include other agents such as emulsifiers, suspending agents, dispersing agents, solubilisers, stabilisers, buffering agents, wetting agents, preserving agents, etc. The compositions may be sterilised by conventional sterilisation techniques. Solutions containing the complexes may be stabilised, for example by the addition of agents such as viscosity modifiers, emulsifiers, solubilising agents, etc.

Preferably, the pharmaceutical compositions for use in the invention will be used in the form of an aqueous suspension of the microbubble complexes in water or a saline solution, e.g. phosphate-buffered saline. The complexes may be supplied in the form of a lyophilised powder for reconstitution at the point of use, e.g. for reconstitution in water, saline or PBS.

The methods herein described involve administration of a pharmaceutically effective amount of the composition which contains the microbubble complexes. The microbubble complexes may then be allowed to distribute to the desired portion or target area of the body prior to activation. Once administered to the body, the target area is exposed to ultrasound at a frequency and intensity to achieve the desired therapeutic effect. A typical activation procedure may involve a two-step process in which the microbubbles are first ruptured by focused ultrasound thereby releasing the sonosensitiser which is then able to penetrate the desired target tissue (e.g. tumour). Subsequent sono-activation of the sonosensitiser within the target cells results in production of singlet oxygen which can oxidise various cell components such as proteins, lipids, amino acids and nucleotides thereby destroying the target cells. Whilst it is envisaged that activation of the sonosensitiser will typically take place subsequent to its delivery (i.e. following burst of the microbubbles to release the sonosensitiser), delivery of the complex and activation of the sonosensitiser may nevertheless be simultaneous.

Alternatively, any of the methods herein described may involve exposure of the target area in the body to ultrasound during administration of the composition which contains the microbubble complexes, i.e. administration of the microbubble complexes and delivery of ultrasound may be carried out simultaneously. Where the half-life of the microbubble complex is low, this can avoid the situation in which a significant proportion may be removed before the target area receives the ultrasound.

The effective dose of any of the compositions herein described will depend on the nature of the complex, the mode of administration, the condition to be treated, the patient, etc. and may be adjusted accordingly.

The frequency and intensity of the ultrasound which may be used can be selected based on the need to achieve selective destruction of the microbubble at the target site and may, for example, be matched to the resonant frequency of the microbubble. Ultrasound frequencies will typically be in the range 20 kHz to 10 MHz, preferably 0.1 to 2 MHz. Ultrasound may be delivered as either a single frequency or a combination of different frequencies. Intensity (i.e. power density) of the ultrasound may range from about 0.1 W/cm² to about 1 kW/cm², preferably from about 1 to about 50 W/cm². Treatment times will typically be in the range of 1 ms to 20 minutes and this will be dependent on the intensity chosen, i.e. for a low ultrasound intensity the treatment time will be prolonged and for a higher ultrasound intensity the treatment time will be lower. Ultrasound may be applied in continuous or pulsed mode and may be either focused or delivered as a columnar beam.

Any radiation source capable of producing acoustic energy (e.g. ultrasound) may be used in the methods herein described. The source should be capable of directing the energy to the target site and may include, for example, a probe or device capable of directing energy to the target tissue from the surface of the body.

In cases where the ultrasound frequencies and/or intensities that are needed to achieve cavitation (or microbubble destruction) and those required to cause sonosensitiser activation are different, these different sets of ultrasound parameters (frequency/intensity) may be applied simultaneously or in a two (or multiple)-step procedure.

Whilst the various methods and uses according to the invention are primarily described herein in the context of administration of a "ready-to-use" microbubble complex, in an alternative embodiment a precursor of the complex may be administered. The term "precursor" as used herein is intended to refer to a precursor for the microbubble complex which is converted *in vivo* to it and is thus essentially equivalent thereto. Thus, for example, the term "precursor" encompasses nanoemulsions or nanodroplet formulations which are capable of conversion to the desired microbubble complex either *in vivo* or during administration. In one embodiment, such precursors are capable of conversion to the desired complex upon accumulation in the target tissue (e.g. tumour tissue). Following distribution to the target tissue or cells, the droplet-to-bubble transition may be triggered by methods which include ultrasound. Alternatively, the step of administration of a precursor of the complex may itself induce formation of a microbubble complex according to the invention. For example, where the precursor takes the form of a nanoemulsion, droplet-to-bubble transition may be induced by injection through a fine gauge needle or by subjecting the preparation to an appropriate phase transition stimulus, e.g. heat. Direct injection of suitable nanoemulsions into target cells or tissues, for example into tumours, and phase transition *in situ* forms a further aspect of the invention.

As will be appreciated, in any of the compositions, methods or uses herein described, any reference to a microbubble complex according to the invention may be replaced by a suitable "precursor" as defined herein.

Nanoemulsions or nanodroplet formulations for use as microbubble complex precursors according to the invention may be produced by appropriate modification of methods and procedures known in the art, for example those disclosed by Rapoport *et al.* (supra). In such formulations, the cores of nanoemulsion droplets, which may be formed by a liquid perfluorocarbon (e.g. a perfluoroalkane), are encased by walls of suitable polymeric, protein or lipid shell materials (e.g. any of the polymers described herein in relation to the microbubble complexes). Linkage of the shells of the nanodroplets to a sonosensitiser(s), a chemotherapeutic agent(s) and/or a linking group(s) as herein described may be achieved using conventional methods and include any of those described above for attaching such moieties to a pre-formed microbubble. The exact method used will be dependent on the exact nature of the shell material, the sonosensitiser, chemotherapeutic agent and/or linking group, specifically the nature of any pendant functional groups. If necessary, either the shell and/or the sonosensitiser, chemotherapeutic agent and/or linking group may be functionalised, e.g. to include reactive functional groups which may be used to couple the moieties. Suitable reactive groups include acid, hydroxy, carbonyl, acid halide, thiol and/or primary amine. In one embodiment the shell may be functionalised with biotin and then bound to avidin to subsequently facilitate binding of a biotinylated linking group. Where it is desired that the formed microbubble will contain oxygen gas, the perfluorocarbon may act as a carrier for the oxygen in liquid form. Following formation of the complex, the perfluorocarbon liquid is saturated with oxygen which subsequently vaporises to form oxygen gas.

The invention will now be described further with reference to the following nonlimiting Examples and the accompanying drawings in which:
**Figure 1** is a schematic representation of the MB-RB conjugate and the bilateral tumour model used in Example 3. Animals received a tail-vein injection of the MB-RB conjugate with ultrasound applied to the treated tumour (+) using the parameters described in Example 3. For the combined SDT / PDL-1 inhibitor experiment, the PDL-1 inhibitor was administered IP 2 hours before the MB-RB + US treatment.
**Figure 2** shows a plot of % tumour growth against time for mice bearing bilateral KPC pancreatic tumours where the right-hand-side tumour was treated with the MB-RB conjugate + US (PRIMARY TUMOUR) while the left-hand-side tumour remained untreated (OFF-TARGET TUMOUR). [MB] = 1.6 x 10⁹; [RB] = 2.15 ± 0.42 mg/kg. n = 5; *p ≤ 0.05, **p ≤ 0.01 for comparison to untreated animals
**Figure 3** shows a plot of % tumour growth for the OFF-TARGET tumour in mice bearing bilateral KPC pancreatic tumours that received (i) no treatment; (ii) an IP injection of PDL-1 inhibitor; (iii) MB-RB conjugate + US at the primary tumour (SDT); and (iv) an IP injection of PDL-1 inhibitor 2 hours before treatment of the primary tumour with the MB-RB conjugate + US (PDL-1 + SDT). [MB] = 1.6 x 10⁹; [RB] = 2.15 ± 0.42 mg/kg; [PDL-1] = 10 mg/kg. Animals treated on days 0, 4 & 7. *p ≤ 0.05, **p ≤ 0.01 for PDL-1 + SDT v SDT.
**Figure 4** shows a plot of average tumour weight for tumours extracted from animals on day 11 following initial treatment.
**Figure 5** shows the fold change in expression of cytotoxic T-cells (CD45+ve, CD3+ve, CD8a+ve) extracted from tumours on day 11 following initial treatment.
**Figure 6** shows a schematic representation of PTX/GEM/RB-MB.
**Figure** 7 is a schematic representation of a) O₂MB-PTX/DOX and b) O₂MB-PTX/RB.

### Examples

### Example 1 - Synthesis of biotin-Rose Bengal

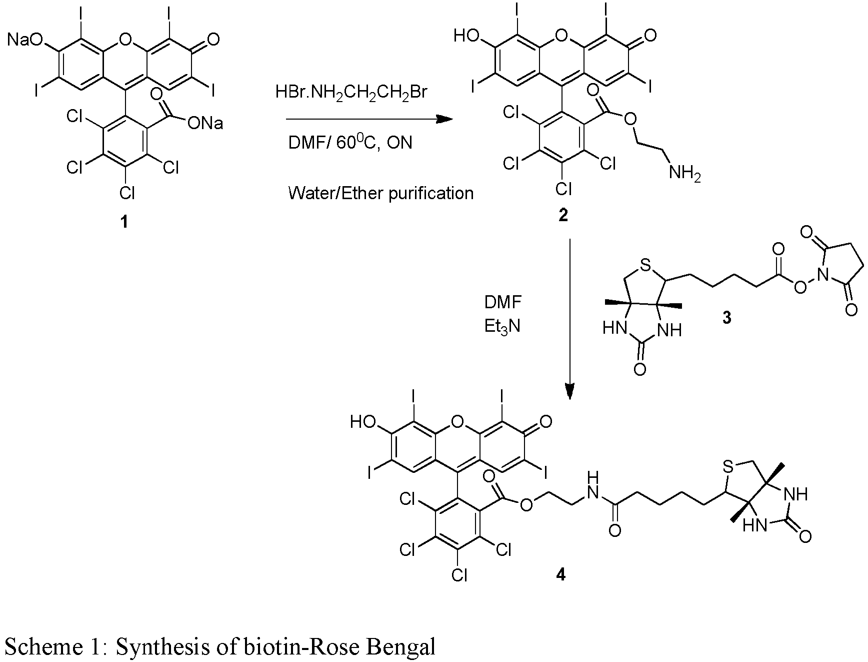

Biotin functionalised Rose Bengal (4) was prepared as described in McEwan et al. (J Control Release. 2015; 203, 51 -6).

### Example 2 - Synthesis of O₂MB-Rose Bengal conjugate

Avidin functionalised lipid stabilised microbubbles (MBs) were prepared by first dissolving 1,2-dibehenoyl-sn-glycero-3-phosphocholine (DBPC) (4.0mg, 4.44µmol), 1,2-distearoyl-sn-glycero-3-phosphoethanolamine-N-[methoxy(polyethylene glycol) -2000] (DSPE-PEG(2000)) (1.35mg, 0.481umol) and DSPE-PEG (2000)-biotin (1.45mg, 0.481µmol) in chloroform to achieve a molar ratio of 82:9:9. The solvent was removed under vacuum at room temperature to yield a translucent film. The dried lipid film was reconstituted in 3mL of PBS (pH 7.4 ± 0.1): propylene glycol:glycerol (8:1:1 v/v) mixture and heated in a water bath at 80°C under magnetic stirring for 30 min. The suspension was then sonicated using a Microson ultrasonic cell disrupter at an amplitude of 22% for 30 seconds. The headspace of the vessel was then filled with perfluorobutane (PFB) gas and the solution was sonicated at an amplitude of 90% for 30 seconds to form the MBs. The MBs were immediately placed on ice for 10 minutes followed by centrifugation at 700 rpm for 5 min and removal of the subnatant. An additional 2mL of PBS (pH 7.4 ± 0.1): propylene glycol:glycerol (8:1:1 v/v) mixture was added to the MB cake followed by an aqueous solution of avidin (500µL, 5mg/mL). The MB suspension was then stirred for 5 min on a rotary shaker (150rpm, 0°C) followed by centrifugation (700 rpm) to remove excess avidin.

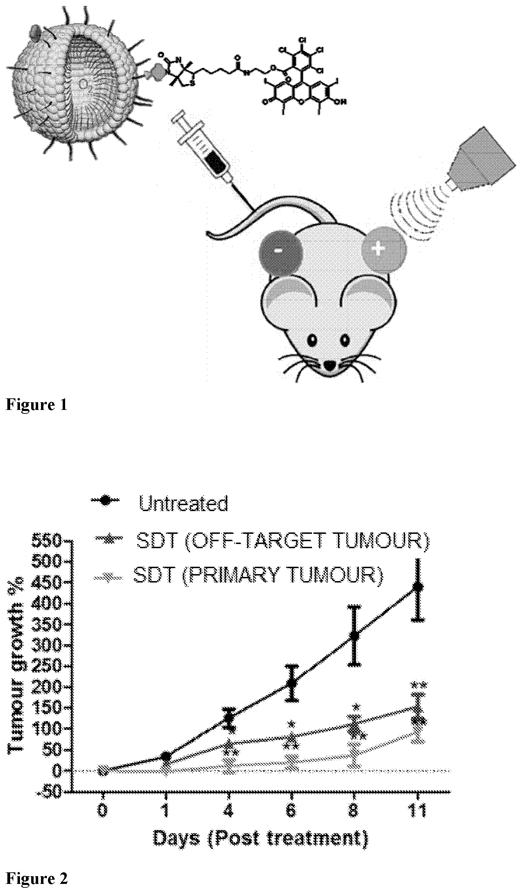

To prepare Rose Bengal (RB) functionalised O₂MBs (as shown in Figure 1), a saturated solution of biotin-RB (4) (1mL, 5mg/mL) in PBS (5% DMSO) was added to avidin-functionalised MBs. The suspension was mixed for 5 min on a rotary shaker (150rpm, 0°C). The subnatant was removed and replaced with 2 mL of PBS (pH 7.4 ± 0.1): propylene glycol:glycerol (8:1:1 v/v) mixture. This washing process was repeated 3 times to remove any unbound material. The MBs were then sparged with medical grade oxygen gas prior to use.

The MB number was determined by withdrawing 10 µL samples of the MB conjugate and diluting in 90 µL of PBS (pH 7.4 ± 0.1) followed by analysis using a haemocytometer (Bright-Line, Hausser Scientific, Horsham, PA, USA).

### Example 3 - In vivo studies

### General:

A mouse pancreatic cancer cell line (T110299), derived from a primary pancreatic tumour of a genetically modified KPC mouse model (Duewell et al., Oncoimmunology (2015) 14; 4(10): e1029698) was used to establish bilateral tumours in the dorsum of immunocompetent BALB/C mice in order to monitor effects at both treated and untreated tumours. When tumours reached a certain size (above 100mm³), the mice were treated with a suspension of O₂MB-RB administered via tail vein injection. The right-hand-side tumour was designated the treated tumour (or "primary tumour") and the left-hand-side tumour the "off-target tumour". During injection, low intensity ultrasound was directed at the target tumour to disrupt the bubbles and activate SDT. A second ultrasound treatment was administered 30 min after the injection to activate the Rose Bengal. These parameters induce microbubble inertial cavitation and maximise SDT mediated generation of ROS. Tumour growth was measured every 2 days and blood samples taken before and 1, 3 and 24 hours following treatment to determine any changes in immune response. Using the same animal model, mice were initially treated with an IP injection of a PDL-1 inhibitor. Two hours later, mice were treated with UTMD mediated SDT and monitored to determine the therapeutic benefit of the combined treatment.

### UTMD mediated SDT treatment using O₂MB-RB conjugate:

All animals employed in this study were treated in accordance with the licenced procedures under the UK Animals (Scientific Procedures) Act 1986. KPC cells were maintained in D-MEM high glucose, 10% Foetal Bovine Serum, 1% L-Glutamine and 1% non-essential amino acids. KPC cells (5x10⁵ per tumour) were sub-cutaneously injected in a 100µl suspension of 1:1 Matrigel and media into the right and left flank of C57BL/6JOlaHsd mice. Bilateral tumours formed and were palpable within 1 week and measured using Vernier callipers. Tumour volume was calculated by (width x width x length)/2. When tumours reached an average size of 119mm³ ± 11.5, the animals were divided into 2 groups (n = 5). Group 1 received no treatment and group 2 received a 100 µl tail intravenous injection of the O₂MB-RB conjugate prepared in Example 2 (MB=1.6 x 10⁹; RB=2.15 ± 0.42 mg/kg) and ultrasound was delivered to the right hand tumour (SDT; Primary Tumour) during injection and for a period of 3.5 minutes. Ultrasound was delivered using a Sonidel SP100 sonoporator (3.5 W cm⁻², 1 MHz, 30% duty cycle, and PRF = 100 Hz; PNP = 0.48 MPa; MI = 0.48; 3.5 minutes) (see Figure 1). The left hand side tumour in group 2 was designated as the SDT; Off Target Tumour. Treatments were repeated on days 4 and 7. Results are presented in Figure 2.

### UTMD mediated SDT treatment using O₂MB-RB conjugate in combination with immune checkpoint inhibitors:

PDL-1 inhibitor: *InVivoMAb* anti-mouse PDL-1 (B7-H1) - Clone: 10F.9G2; Catalog # BE0101 (supplier 2BScientific).

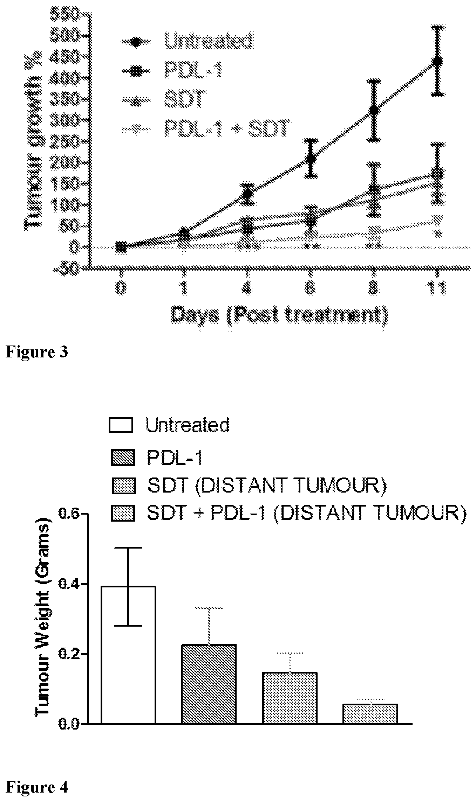

For MB mediated SDT + anti-PDL-1 treatment, bilateral tumours were established in a further four groups (n = 5) of animals as described above. When tumours reached an average of 150mm³ ± 11.8, the animals were treated as follows: group 1 received no treatment; group 2 received an intraperitoneal injection of PDL-1 inhibitor (10mg/kg), group 3 received a 100µl tail intravenous injection of O₂MB-RB conjugate (MB=1.6 x 10⁹; RB=2.15 ± 0.42 mg/kg) and ultrasound was delivered to the primary tumour during injection and for a period of 3.5 minutes as described above. Group 4 received an intraperitoneal injection of PDL-1 inhibitor (10mg/kg) and 2 hours later animals were treated with a 100µl tail intravenous injection of O₂MB-RB conjugate (MB=1.6 x 10⁹; RB=2.15 ± 0.42 mg/kg) and ultrasound was delivered to the primary tumour during injection and for a period of 3.5 minutes. Tumours were measured daily for 11 days. Results are presented in Figure 3. Treatments were repeated on days 4 and 7. On Day 11, animals were sacrificed, tumours were excised and the tumour weights recorded. Results are presented in Figure 4.

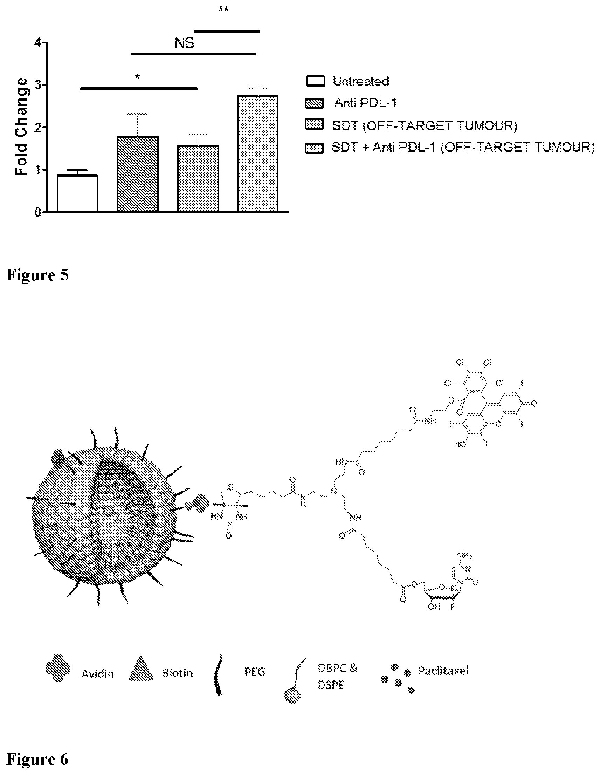

A single cell suspension was obtained from the tumours by homogenising the tumour, adding 5 ml of 4 % FCS in RPMI and 160 µl (30 mg/ml) collagenase type II (Gibco, 17101-015) and 50 µl (2µ g/ml) DNAse and stirring for 15 minutes. A further 160 µl (30 mg/ml) collagenase was added and stirred for a further 15 minutes at room temperature. The suspension was filtered through a 100µm filter, centrifuged at 1700 rpm for 5 minutes, re-suspended in 1ml Red Lysis buffer and incubated for 10 minutes at room temperature. The remaining cells were centrifuged, the supernatant removed and washed twice in ice cold PBS. Cells were incubated in fluorochrome conjugated antibodies specific for CD45 (0.125µg/test, eBioscience), CD3 (0.5µg/test, eBioscience), CD8a (0.25µg/test, eBioscience). Red blood cells were removed using multi-species RBC lysis buffer as per the manufacturer's instructions (eBioscience). Cytotoxic T cells were identified as CD45+CD3+CD8a+ cells. Cells were acquired using a Gallios (Beckman Coulter) and analysed using FlowJo software. Results are presented in Figure 5.

### Example 4 - Synthesis of Biotin-Gemcitabine (Biotin-Gem) and Biotin-Gemcitabine-Rose Bengal (Biotin-Gem-RB) conjugates

### 4.1 Synthesis of biotinylated Gemcitabine (Biotin-GEM):

Biotinylated Gemcitabine was synthesised according to scheme 2. The protocol is provided below.

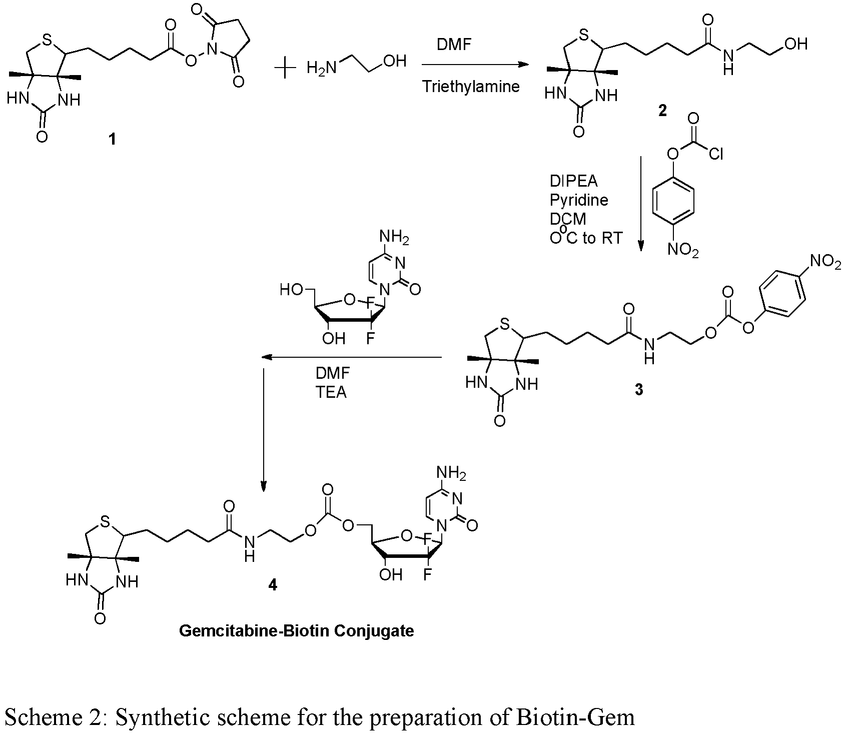

### Synthesis of (2R, 3R, 5R)-5-(4-amino-2-oxopyrimidin-1(2H)-yl)-4, 4-difluoro-3 hydroxytetrahydrofuran-2-yl)methyl (2-(5-(2-oxohexahydro-1H-thieno[3,4-dfimidazol-4-yl)pentanamido)ethyl) carbonate (4):

Compound 2 was prepared following a literature procedure (see McEwan et al. Biomaterials. 2016: 80, 20-32). To a DCM (10 mL) solution of **2** (0.28g, 0.9 mmol), 4-nitrophenyl chloroformate (0.59g, 2.9 mmol), DIPEA (0.50g, 3.9 mmol) and a catalytic amount of pyridine were added at 0°C and stirred for 24 hrs at room temperature. Then the reaction mixture was concentrated to dryness *in vacuo.* The crude residue containing 3 was dissolved in 20 mL DMF. To this solution, Gemcitabine (0.88g, 2.9 mmol) in DMF (5 mL) and TEA (1 mL) were added and the mixture stirred for a further 24 hrs. After completion of reaction (monitored by TLC), excess diethyl ether (200 mL) was added to the reaction mixture and stirred for 45 min. The yellowish oil thus obtained was separated and washed three times with cold diethyl ether (50 mL x 3). The crude compound was purified by PTLC using DCM/MeOH (9 : 1) as eluent to afford the target compound 4 (0.12g, 22% yield).

¹H NMR (DMSO-d₆): δ 7.99-7.91 (m, 3H, CH, NH₂), 6.41-6.33 (m, 1H, CH), 6.12-6.01 (m, 3H, CH, NH X 2), 4.30-4.16 (m, 1H, CH), 4.19-4.12 (m, 3H, CH, CH₂), 3.90-3.75 (m, 2H, CH₂), 3.69-3.58 (m, 2H, CH₂), 3.12-3.09 (m, 1H, CH), 2.93-2.88 (m, 2H, CH₂), 2.83 (brs, 1H, OH), 2.82-2.77 (m, 2H, CH X2), 2.72 (brs, 1H, NH),2.49-2.04 (m, 2H, CH₂), 1.49-1.28 (m, 6H, CH₂ X 3).

¹³C NMR (DMSO-d₆): 175.0 (C=O), 166.3 (C), 165.5 (C=O), 156.3 (C=O), 156.1 (C=O), 141.3 (CH), 125.3 (C), 95.2 (CH), 79.2 (CH), 67.2 (CH), 61.9(CH), 60.2 (OCH₂), 55.5 (OCH₂), 39.6 (CH), 37.8 (CH₂), 35.2 (CH), 28.3 (CH₂), 28.0 (CH₂), 25.3 (CH₂). ESI-MS: cald for C₂₂H₃₀F₂N₆O₈S, 576.18; found 577.2 (M + H).

### 4.2 Synthesis of Biotin-Gem-RB:

Biotin-Gem-RB was synthesised according to Scheme 3. The protocols for each intermediate are provided below.

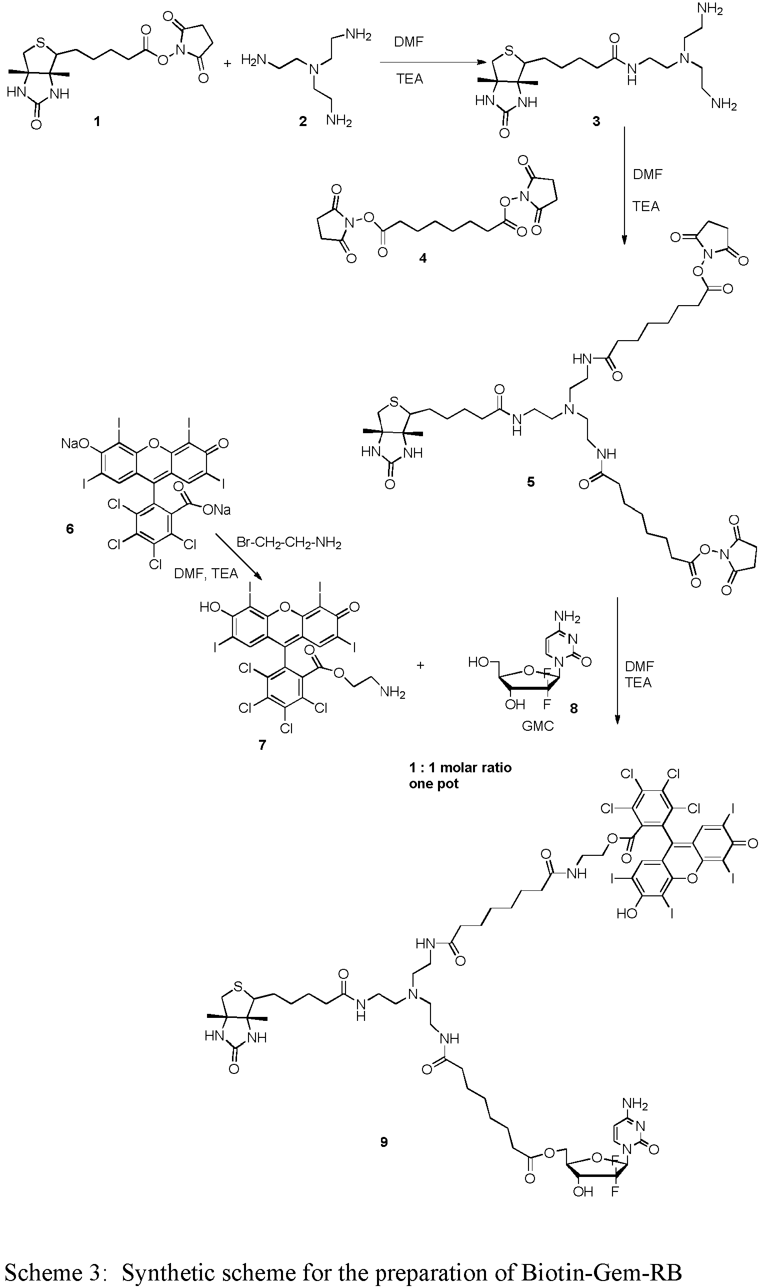

### Synthesis of N-(2-(bis(2-aminoethyl)amino)ethyl)-5-(2-oxohexahydro-1H-thieno[3,4-d]imidazol-4-yl)pentanamide (3):

To a stirred solution of Biotin-NHS (0.5g, 1.5 mmol) and TEA (catalytic amount) in anhydrous DMF (10 mL), a solution of tris(2-aminoethyl)amine ( 0.22g, 1.5 mmol) in 5mL of DMF was added. The reaction mixture was stirred at 0°C under argon atmosphere. After 2 hr of stirring, another volume of TEA (catalytic amount) was added and the reaction mixture was allowed to stir overnight at room temperature. After completion of the reaction (by TLC), the excess DMF was removed under reduced pressure keeping the temperature below 45°C and the white gummy liquid thus obtained was poured into excess diethyl ether (200 mL) and filtered. The crude product was purified by column chromatography on basic (TEA) silica gel (MeOH: DCM 1:9 to 3:7) to give 3 (0.33g, 61% yield) as a white semi solid.

¹H NMR (DMSO-d₆): δ 7.94 (brs, 1H, NH), 6.42 (brs, 1H, NH), 6.35 (brs, 1H, NH), 4.49 (brs, 4H, NH₂ X 2), 4.29 (s, 1H, CH), 4.12 (s, 1H, CH), 3.07-3.02 (m, 6H, CH₂ X 3), 2.88-2.82 (m, 1H, CH), 2.44-2.06 (m, 10H, CH₂ X 5), 1.59-1.48 (m, 4H, CH₂ X 2), 1.47-1.29 (m, 2H, CH₂).

ESI-MS: cald for C₁₆H₃₂N₆O₂S, 372.23; found 373.31 (M +H).

### Synthesis of bis(2,5-dioxopyrrolidin-1-yl) 8,8'-((((2-(5-(2-oxohexahydro-1H-thieno[3, 4-d]imidazol-4-yl)pentanamido)ethyl)azanediyl)bis(ethane-2,1-diyl))bis(azanediyl))bis(8-oxooctanoate) (5):

Compound 3 (0.5g, 1.3 mmol) was dissolved in 10 mL anhydrous DMF in the presence of TEA (catalytic amount) and bis(2,5-dioxopyrrolidin-1-yl) octanedioate **(4,** 1g, 2.7mmol) was added. The reaction mixture was stirred at room temperature for 24 hrs under argon atmosphere. After completion of the reaction (by TLC), excess diethyl ether (200 mL) was added to the reaction mixture. The white precipitate thus obtained was filtered and washed three times with cold diethyl ether (50 mL x 3). The crude product was purified by column chromatography on basic (TEA) silica gel (MeOH: CHCl₃ 2:8 to 5:5 v/v) to give 5 (0.83g, 71% yield) as a low melting white solid.

¹H NMR (DMSO-d₆): δ 7.94 (brs, 2H, NH X 2), 7.67 (brs, 1H, NH), 6.41 (brs, 1H, NH), 6.34 (brs, 1H, NH), 4.29 (s, 1H, CH), 4.12 (s, 1H, CH), 3.06-3.04 (m, 3H, CH and CH₂), 2.88-2.72 (m, 6H, CH₂ X 3), 2.71-2.63 (m, 8H, CH₂ X 4), 2.45-2.34 (m, 6H, CH₂ X 3), 2.20-2.06 (m, 10H, CH₂ X 5), 1.60-1.21 (m, 22H, CH₂ X 11).

¹³C NMR (DMSO-d₆): 172.5 (C=O), 170.7 (C=O), 163.1 (C=O), 162.7 (C=O), 61.4 (CH), 59.6 (CH), 55.8 (CH₂), 53.9 (NCH₂), 39.9 (CH₂), 39.8(CH₂), 39.6(CH₂), 37.3(CH₂), 36.2(CH₂), 35.6(CH₂), 31.2 (CH₂), 28.7(CH₂), 28.5(CH₂), 25.8(CH₂), 25.7(CH₂), 25.6(CH₂).

ESI-MS: cald for C₄₀H₆₂N₈O₁₂S, 878.4; found 901.3 (M +Na salt).

### Synthesis of ((2R, 3R, 5R)-5-(4-amino-2-oxopyrimidin-1(2H)-yl)-4, 4-difluoro-3-hydroxytetrahydrofuran-2-yl)methyl 4,11,19-trioxo-15-(2-(5-(2-oxohexahydro-1H-thieno[3,4-d]imidazol-4 yl)pentanamido)ethyl)-1-((2,3,4,5-tetrachloro-6-(6-hydroxy-2,4,5,7-tetraiodo-3-oxo-3H-xanthen-9-yl)benzoyl)oxy)-3,12,15,18-tetraazahexacosan-26-oate (9):

To a DMF (anhydrous, 10 mL) solution of 5 (0.4g, 0.45 mmol) GMC-hydrochloride **(8,** 0.136g, 0.45 mmol) and TEA (0.5 mL) were added at 0°C and stirred for 24 hrs at room temperature under argon atmosphere. After completion of the reaction (monitored by GC-MS), Rose Bengal amine 7 (prepared according to McEwan et al. J. Control Release. 2015; 203, 51-56), (0.43g, 0.45mmol in DMF (5 mL)) and TEA (0.5 mL) were added to the reaction mixture and continued to stir for 24 hr. The progress of the reaction was monitored by mass spec analysis of the crude reaction mixture. After completion of the reaction, excess diethyl ether (200 mL) was added to the solution and stirred for 30 min. The pink red precipitate thus obtained was filtered and washes several times with cold diethyl ether (100 mL), ethyl acetate (100 mL), acetone-water mixture (10%, v/v, 100 mL) and finally with ethyl acetate-hexane mixture (50%, v/v, 100 ml) respectively to afford a pink red powder of compound 9 (0.26g, 30% yield).

¹H NMR (DMSO-d₆): δ 7.95 (brs, 2H, NH₂), 7.69 (s, 1H, CH, aromatic proton), 7.68 (s, 1H, CH, aromatic proton), 7.37(s, 1H, CH), 7.32 (brs, 4H, NH X 4), 6.89 (s, 1H, CH), 6.42 (brs, 1H, NH), 6.35 (brs, 1H, NH), 6.22 (d, *J =* 5.5 Hz, 1H, CH), 6.13 (brs, 1H, NH), 5. 78-5.77 (m, 1H, CH), 5.19 (s, 1H, CH X 2), 4.9 (brs, 1H, OH), 4.30 (s, 2H, -OCH₂), 4.13 (s, 2H, -OCH₂), 3.79-3.60 (m, 3H, CH, CH₂), 3.39-3.32 (m, 2H, CH₂), 3.07 (brs, 6H, N-NH*CH₂* X 3), 2.94-2.84 (m, 6H, NCH₂ X 3), 2.81 (brs, 1H, OH), 2.45-2.46 (m, 3H, CH, CH₂), 2.17-2.06 (m, 10H, CH₂ X 5), 1.60-1.10 (m, 22H, CH₂ X 11).

¹³C NMR (DMSO-d₆): 171.8 (C=O, C), 165.98 (C=O), 163.2 (C=O), 162.7 (C=O), 159.3 (CH), 155.0 (C=O), 150.5 (C), 145.8 (C), 141.2 (CH), 131.0 (C), 128.7 (C), 123.5 (C), 116.2 (C), 95.0 (CH), 80.9 (C), 69.3 (CH), 61.5 (C), 59.6 (CH₂), 59.4 (CH), 55.8 (CH), 51.7 (CH₂), 45.8 (CH₂), 40.2 (CH₂), 37.3 (CH₂), 37.05 (CH₂), 36.2 (CH₂), 35.5 (CH₂), 31.0 (CH₂), 28.7 (CH₂), 28.5 (CH₂), 28.2 (CH₂), 25.6 (CH₂). ESI-MS: cald for C₆₃H₇₂Cl₄F₂I₄ N₁₉O₁₅S, 1925.98; found 1925.90 (M -H).

### Example 5 - Preparation of Avidin-functionalised Paclitaxel (PTX) loaded Microbubbles (MBs)

### 5.1 Preparation of lipid stabilised MBs with PTX incorporated within the shell (PTX-MB):

Avidin functionalised lipid stabilised microbubbles with PTX hydrophobically incorporated in the shell were prepared by first dissolving the lipids 1,2-dibehenoyl-sn-glycero-3-phosphocholine (DBPC) (4.0mg, 4.43umol), 1,2-distearoyl-sn-glycero-3-phosphoethanolamine-N-[methoxy(polyethylene glycol)-2000] (DSPE-PEG(2000)) (1.35mg, 0.481µmol) and DSPE-PEG (2000)-biotin (1.45mg, 0.481µmol) in chloroform to achieve a molar ratio of 82:9:9. To this solution was added paclitaxel (5mg, 5.86µmol) dissolved in chloroform. The solution was heated at 40°C for 30 minutes until the chloroform had evaporated. The dried lipid film was reconstituted in 3mL of Ungers solution (PBS, Glycerol, Propylene glycol (8:1:1 volume ratio)) and heated on a water bath at 75°C for 30 minutes. The suspension was then sonicated using a Microson ultrasonic cell disrupter at an amplitude of 22% for 1 minute to fully incorporate the lipids with paclitaxel. The suspension was then sparged with PFB gas whilst sonicating the suspension at an amplitude of 89% for 1 min to form the microbubble suspension. The MBs were then cooled on ice for 10 minutes followed by centrifugation at 700 rpm for 5 min to remove the excess lipids/paclitaxel present in the liquid below the bubble cake. The cake was then washed with 2mL of Ungers solution followed by the addition of an aqueous solution of avidin (500µL, 2.5mg/mL). The suspension was then stirred for 5 min followed by centrifugation (700 rpm) to remove excess avidin. The MB cake was then washed again with 2mL of Ungers solution, centrifuged (700 rpm) and the MBs isolated.

### 5.2 Loading of Biotin-RB, Biotin-Gem and Biotin-Gem-RB onto the surface of avidin functionalised PTX-MBs:

A solution containing either Biotin-RB, Biotin-Gem, or Biotin-Gem-RB (500µL, 5mg/mL), prepared in a DMSO: Ungers solution (10:90 v/v) was added to 2 mL of PTX-MBs (2.0 x 10⁸ MB/mL). The suspension was then mixed for 5 min using a rotary shaker followed by centrifugation (700 rpm) for 5 min to remove excess ligand. This coupling process was repeated one more time. The final microbubble cake was suspended in 2mL of Ungers solution. The microbubbles were either used directly or oxygenated by sparging the suspension with oxygen gas for 2 min immediately prior to use.

### Example 6 - Preparation of Avidin functionalised PTX-MBs carrying Biotin-RB, Biotin-Gem and Biotin-Gem-RB

### 6.1 Loading of Biotin-RB, Biotin-Gem and Biotin-Gem-RB onto the surface of avidin functionalised PTX-MBs:

A saturated aqueous solution containing either Biotin-RB, Biotin-Gem or Biotin-Gem-RB (1mL, 5mg/mL) was added to 2 mL of PTX-MBs or PTX-free MBs (6.72 x 10⁸ MB/mL). The suspension was mixed for 5 min (0°C) followed by centrifugation (700 rpm) for 3 min to remove excess ligand. The MB cake was then washed a further 3 times with PBS solution. The final microbubble cake was suspended in 2mL of PBS solution. The microbubbles were either used directly or oxygenated by sparging the suspension with oxygen gas for 2 min immediately prior to use. The final microbubble number was determined on a haemocytometer using an optical microscope. A schematic representation of the PTX/GEM/RB-MB is shown in Figure 6.

### Example 7 - Preparation of Paclitaxel (PTX) loaded Microbubbles (MBs) carrying Biotin-Doxorubicin (Biotin-Dox) or Biotin-Rose Bengal (Biotin-RB) conjugates

### 7.1 Reagents and Materials:

1,2-dibehenoyl-*sn*-glycero-3-phosphocholine (DBPC) and 1,2-distearoyl-sn-glycero-3-phosphoethanolamine-N-[methoxy(polyethylene glycol)-2000] (DSPE-PEG(2000)) and DSPE-PEG(2000)-biotin were purchased from Avanti Polar Lipids (Alabaster, Alabama, USA). Oxygen gas was purchased from BOC Industrial Gases UK and perfluorobutane (PFB) was purchased from Apollo Scientific Ltd. Phosophate Buffered Saline (PBS) was purchased from Gibco, Life Technologies, UK. Glycerol and propylene glycol (1 kg, hydrolysed) were purchased from Sigma Aldrich (UK). Optical microscope images were obtained using a Leica DM500 optical microscope. Rose Bengal sodium salt, NHS-biotin, gemcitabine, MTT assay kit, avidin, chloroacetic acid, 4-dimethylaminopyridine (DMAP), hydroxybenzotriazole (HOBt), *N,N'*-dicyclohexylcarbodiimide (DCC), anhydrous dimethylformamide (DMF), and ethanol were purchased from Sigma Aldrich (UK) at the highest grade possible. Biotin, di(N-succinimidyl) carbonate and 2-aminoethanol were purchased from Tokyo Chemical Industry UK Ltd.

### 7.2 Synthesis of Biotin-Dox:

Biotinylated Doxorubicin (Biotin-Dox) (3) was synthesised according to scheme 4. The protocol is provided below.

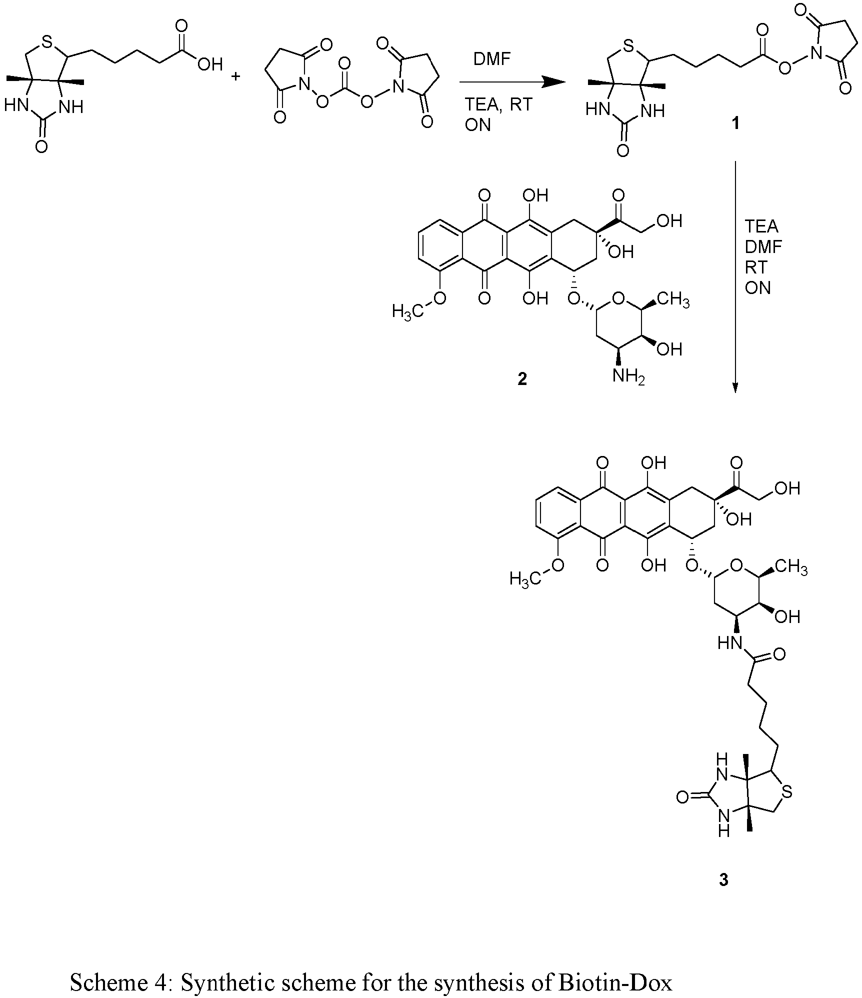

To an ice cold solution of biotin-N-hydroxysuccinimide ester (1, 0.14g, 0.41 mmmol) in DMF (10 ml) was added doxorubicin (2, 0.3g, 0.41mmol) under nitrogen atmosphere. After stirring for 30 min, triethylamine (0.5ml, 2 mmol) was added to this reaction mixture and was allowed to stir for another 12 hrs at room temperature. The reaction was monitored by TLC (Merck Silica 60, HF 254, 20: 80 methanol-dichloromethane v/v). After completion of the reaction, excess diethyl ether (100 ml) was added to the reaction mixture. The red solid thus obtained was filtered and washed three times with diethyl ether (50 ml X 3). This red solid was then subjected to PTLC purification using methanol-dichloromethane (20:80, v/v) as an eluent to obtain 0.25g (Yield = 78%) of 3. An analytical sample was obtained from a recrystallization of this product from ethanol.

¹H NMR (DMSO-d₆)δ :7.84 (d, J = 7.5 Hz, 2H, aromatic), 7.58 (d, J = 7.5 Hz, 1H, aromatic), 6.36 (s, 1H, NH), 6.29 (s, 1H, NH), 5.37 (brs, 1H, OH), 5.22 (brs, 1H, OH), 4.87 (s, 2H, -CH₂-OH), 4.51 (brs, 2H, OH X 2), 4.36-4.33 (m, 1H, CH), 4.25-4.22 (m, 1H, CH), 4.16-4.13 (m, 1H, CH), 3.99 (s, 3H, OCH₃), 3.60-3.58 (m, 1H, CH), 3.55 (brs, 2H, OH X2), 3.10-3.00 (m, 4H, CH₂ X1, CH X 2), 2.88-2.54 (m, 3H, CH₂ X 1, CH), 2.20-2.00 (m, 1H, CH), 1.63-1.50 (m, 4H, CH₂ X 2), 1.42-1.22 (m, 11 H, CH₃ X 1, CH₂ X 4). ¹³CNMR (DMSO-d₆): 177.6, 176.9, 174.8, 166.4, 163.0, 161.2, 153.7, 152.7, 137.4, 132.4, 120.4, 119.4, 99.5, 97.8, 80.15, 75.1, 72.7, 66.4, 61.4, 59.5, 55.7, 47.8, 33.8, 31.9, 28.9, 28.8, 28.5, 28.4, 24.9, 19.8, 17.6, 17.1. ESMS (M-H]: calculated for C₃₇H₄₃I₂N₃O₁₃S = 769.25, found = 767.9.

### 7.3 Preparation of oxygen carrying microbubbles loaded with PTX in the shell and either biotin-Dox or biotin-RB attached to the MB surface:

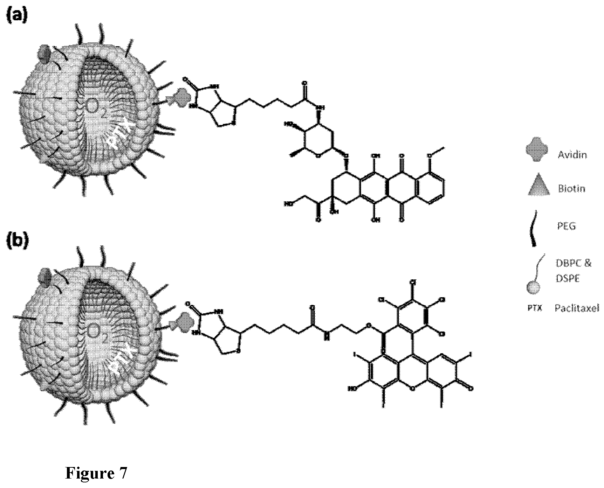

Avidin-functionalised lipid-stabilised microbubbles with PTX hydrophobically incorporated in the shell were prepared by dissolving 1,2-dibehenoyl-sn-glycero-3-phosphocholine (DBPC) (4.0mg, 4.44umol), 1,2-distearoyl-sn-glycero-3-phosphoethanolamine-N-[methoxy(polyethylene glycol) -2000] (DSPE-PEG(2000)) (1.35mg, 0.481umol) and DSPE-PEG (2000)-biotin (1.45mg, 0.481umol) in chloroform to achieve a molar ratio of 82:9:9. To this solution was added paclitaxel (5mg, 5.86µmol) dissolved in chloroform (100uL). The solvent was removed under vacuum at room temperature yielding a translucent film. The dried lipid film was reconstituted in 2mL of a solution containing PBS, Glycerol and Proplyene glycol (8:1:1 volume ratio) and heated in a water bath at 80°C for 30 minutes. The suspension was then sonicated using a Microson ultrasonic cell disrupter at an amplitude of 22% for 30 seconds to fully suspend paclitaxel. The suspension was then sparged with PFB gas whilst sonicating the suspension at an amplitude of 89% for 1 minute to form the microbubbles. The MBs were then cooled on ice for 10 minutes followed by centrifugation at 700 rpm for 3 min and removal of the subnatant to remove the excess lipids/paclitaxel. The cake was then washed a further 2 times before an aqueous solution of avidin (10 mg/mL) was added. The suspension was then stirred for 5 min (0°C) followed by centrifugation (700 rpm) to remove unbound avidin. The MB cake was then washed again and suspended in PBS solution. A saturated aqueous solution containing either Biotin-Dox or biotin-RB (1mL, 5mg/mL) was added to 2 mL of PTX-MBs (7.52 x 10⁸ MB/mL). The suspension was mixed for 5 min (0°C) followed by centrifugation (700 rpm) for 3 min to remove excess ligand. The MB cake was then washed a further 3 times with PBS solution. The final microbubble cake was suspended in 2mL of PBS solution. The microbubbles were oxygenated by sparging the suspension with oxygen gas for 2 min immediately prior to use. The final microbubble number was determined on a haemocytometer using an optical microscope. Figure 7 is a schematic representation of a) O₂MB-PTX/DOX b) O₂MB-PTX/RB.

### Example 8 - Preparation of Biotin-Doxorubicin-Rose Bengal conjugate

A tri-podal Biotin-Doxorubicin-Rose Bengal conjugate (Biotin-Dox-RB) was synthesised according to Scheme 5:

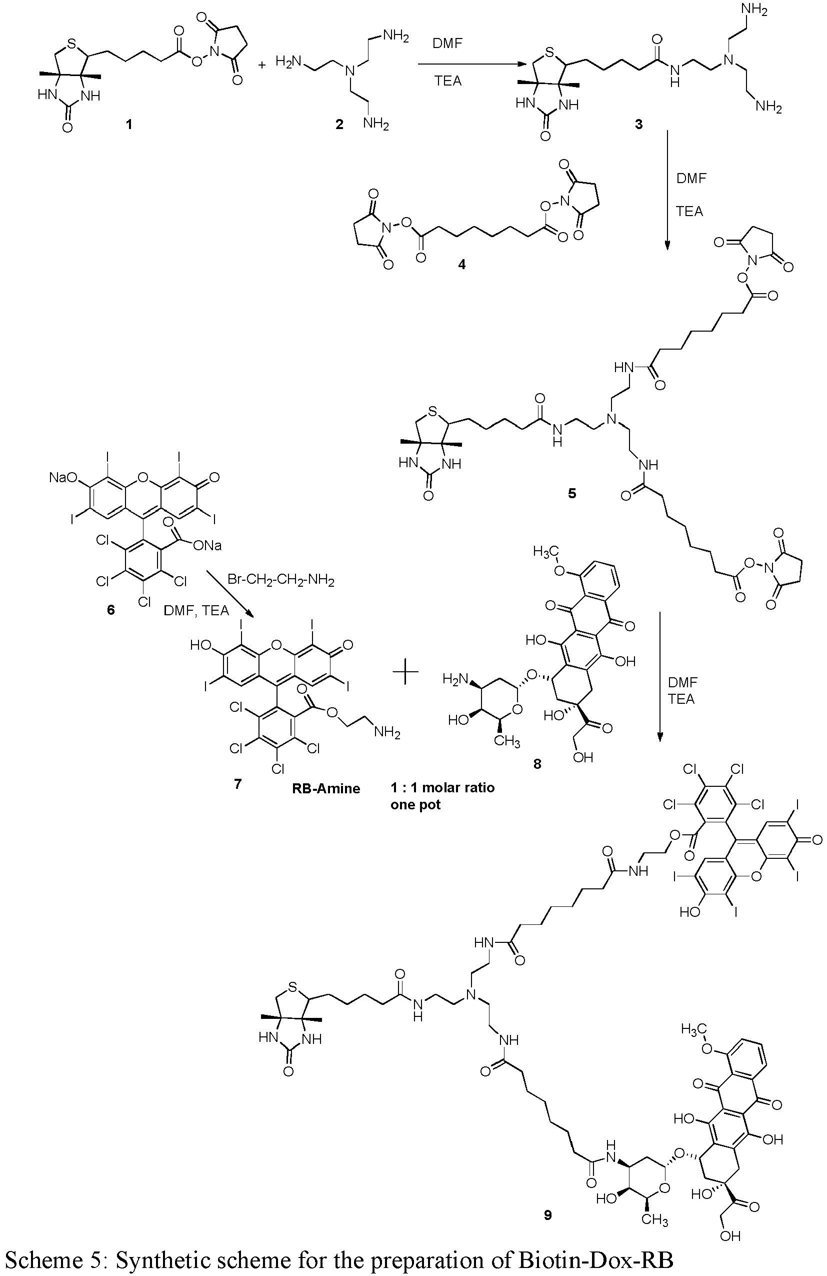

### 8.1 Synthesis of N-(2-(bis(2-aminoethyl)amino)ethyl)-5-(2-oxohexahydro-1H-thieno[3,4-d]imidazol-4-yl)pentanamide (3):

To a stirred solution of Biotin-NHS (0.5g, 1.5 mmol) and TEA (catalytic amount) in anhydrous DMF (10 mL), a solution of tris(2-aminoethyl)amine ( 0.22g, 1.5 mmol) in 5mL of DMF was added. The reaction mixture was stirred at 0°C under argon atmosphere. After 2 hr of stirring, another volume of TEA (catalytic amount) was added and the reaction mixture was allowed to stir overnight at room temperature. After completion of the reaction (by TLC), the excess DMF was removed under reduced pressure keeping the temperature below 45°C and the white gummy liquid thus obtained was poured into excess diethyl ether (200 mL) and filtered. The crude product was purified by column chromatography on basic (TEA) silica gel (MeOH: DCM 1:9 to 3:7) to give 3 (0.33g, 61% yield) as a white semi solid.

¹H NMR (DMSO-d₆): δ 7.94 (brs, 1H, NH), 6.42 (brs, 1H, NH), 6.35 (brs, 1H, NH), 4.49 (brs, 4H, NH₂ X 2), 4.29 (s, 1H, CH), 4.12 (s, 1H, CH), 3.07-3.02 (m, 6H, CH₂ X 3), 2.88-2.82 (m, 1H, CH), 2.44-2.06 (m, 10H, CH₂ X 5), 1.59-1.48 (m, 4H, CH₂ X 2), 1.47-1.29 (m, 2H, CH₂). ESI-MS: cald for C₁₆H₃₂N₆O₂S, 372.23; found 373.31 (M +H).

### 8.2 Synthesis of bis(2,5-dioxopyrrolidin-1-yl) 8,8'-((((2-(5-(2-oxohexahydro-1H-thieno[3,4-d]imidazol-4-yl)pentanamido)ethyl)azanediyl)bis(ethane-2,1-diyl))bis(azanediyl))bis(8-oxooctanoate) (5):

Compound 3 (0.5g, 1.3 mmol) was dissolved in 10 mL anhydrous DMF in the presence of TEA (catalytic amount) and bis(2,5-dioxopyrrolidin-1-yl) octanedioate (4, 1g, 2.7mmol) was added to it. The reaction mixture was stirred at room temperature for 24 hrs under argon atmosphere. After completion of the reaction (by TLC), excess diethyl ether (200 mL) was added to the reaction mixture. The white precipitate thus obtained was filtered and washed three times with cold diethyl ether (50 mL X 3). The crude product was purified by column chromatography on basic (TEA) silica gel (MeOH: CHCl₃ 2:8 to 5:5 v/v) to give 5 (0.83g, 71% yield) as a low melting white solid.

¹H NMR (DMSO-d₆): δ 7.94 (brs, 2H, NH X 2), 7.67 (brs, 1H, NH), 6.41 (brs, 1H, NH), 6.34 (brs, 1H, NH), 4.29 (s, 1H, CH), 4.12 (s, 1H, CH), 3.06-3.04 (m, 3H, CH and CH₂), 2.88-2.72 (m, 6H, CH₂ X 3), 2.71-2.63 (m, 8H, CH₂ X 4), 2.45-2.34 (m, 6H, CH₂ X 3), 2.20-2.06 (m, 10H, CH₂ X 5), 1.60-1.21 (m, 22H, CH₂ X 11). ¹³C NMR (DMSO-d₆): 172.5 (C=O), 170.7 (C=O), 163.1 (C=O), 162.7 (C=O), 61.4 (CH), 59.6 (CH), 55.8 (CH₂), 53.9 (NCH₂), 39.9 (CH₂), 39.8(CH₂), 39.6(CH₂), 37.3(CH₂), 36.2(CH₂), 35.6(CH₂), 31.2 (CH₂), 28.7(CH₂), 28.5(CH₂), 25.8(CH₂), 25.7(CH₂), 25.6(CH₂). ESI-MS: cald for C₄₀H₆₂N₈O₁₂S, 878.4; found 901.3 (M +Na salt).

### 8.3 Synthesis of 26-(((2S,3S,4S,6R)-3-hydroxy-2-methyl-6-(((1S,3S)-3,5,12-trihydroxy-3-(2-hydroxyacetyl)-10-methoxy-6,11-dioxo-1,2,3,4,6,11-hexahydrotetracen-1-yl)oxy)tetrahydro-2H-pyran-4-yl)amino)-4,11,19,26-tetraoxo-15-(2-(5-(2-oxohexahydro-1H-thieno[3,4-d]imidazol-4-yl)pentanamido)ethyl)-3,12,15,18-tetraazahexacosyl 2,3,4,5-tetrachloro-6-(6-hydroxy-2,4,5,7-tetraiodo-3-oxo-3H-xanthen-9-yl)benzoate (9):

To a DMF (anhydrous, 10 mL) solution of 5 (0.4g, 0.45 mmol) Doxorubicinhydrochloride (8, 0.26g, 0.45 mmol) and TEA (0.5 mL) were added at 0°C and stirred for 24 hrs at room temperature under argon atmosphere. After completion of the reaction (monitored by GC-MS), Rose Bengal amine 7 (prepared separately according to literature procedure), 0.46g, 0.45mmol} in DMF (5 mL) and TEA (0.5 mL) were added to the reaction mixture and continued to stir for 24 hrs. The progress of the reaction was monitored by GC-MS analysis of the crude reaction mixture. After completion of reaction, excess diethyl ether (200 mL) was added to the solution and stirred for 30 min. The dark red precipitate thus obtained was filtered and washes several times with cold diethyl ether (100 mL), ethyl acetate (100 mL), acetone-water mixture (10%, v/v, 100 mL) and finally with ethyl acetate-hexane mixture (50%, v/v, 100 ml) respectively to afford a red powder of compound 9 (0.28g, 28% yield).

¹H NMR (DMSO-d₆):7.93 (s, 2H, Ar-CH), 7.90-7.85 (m, 2H, Ar-CH), 7.66 (brs, 5H, NH), 7.45 (s, 1H, 6.39 (brs, 1H, NH), 6.33 (brs, 1H, NH), 5.41-5.39 (m, 1H, CH), 5.19-5.18 (m, 4H, -CH₂ X 2), 4.93-4.71 (m, 3H, CH X 3), 4.55 (s, 3H, -OCH₃),4.27-3.90 (m, 4H, CH X 4), 3.04-2.97 (m, 8H, CH₂ X 4), 2.80-2.77 (m, 2H, CH₂), 2.48-2.43 (m, 8H, CH₂ X 4), 2.03 (brs, 12H, CH₂ X 6), 1.43 (brs, 12H, CH₂ X 6), 1.14-1.11 (m, 13H, CH₂ X 5, CH₃ X 1).

¹³C NMR (DMSO-d₆): 220.1, 177.3, 172.5, 172.2, 171.5, 168.9, 163.2, 162.7, 157.3, 156.6, 154.6, 153.7, 150.3, 138.5, 136.5, 135.6, 133.0, 110.7, 101.9, 97.6, 96.3, 89.2, 79.3, 76.7, 66.8, 63.5, 61.4, 60.5, 59.6, 57.5, 55.8, 53.9, 51.9, 40.4, 40.2, 37.4, 36.5, 36.2, 35.6, 31.2, 28.7, 28.4, 28.3, 25.7.

ESI-MS: cald for C₈₁H₉₀Cl₄I₄ N₈O₂₂S, 2209.12; found 2208.02 (M -H).

### Example 9 - Alternative method for the synthesis of Biotin-Gemcitabine-Rose Bengal conjugate

A Biotin-Gemcitabine-Rose Bengal (Biotin-Gem-RB) conjugate was synthesised according to Scheme 6:

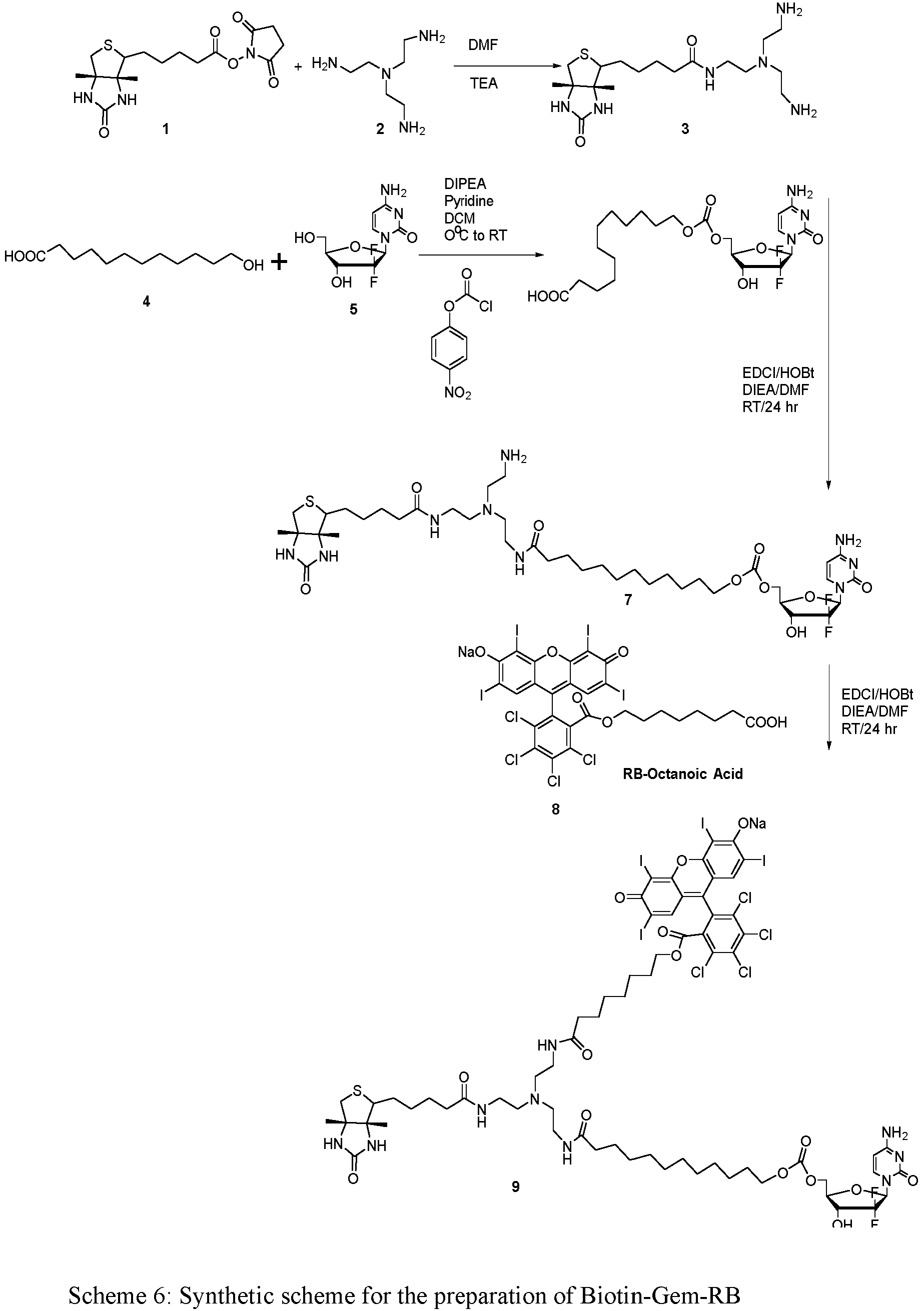

### 9.1 Synthesis of N-(2-(bis(2-aminoethyl)amino)ethyl)-5-(2-oxohexahydro-1H-thieno[3,4-d]imidazol-4-yl)pentanamide (3):

Compound 3 was synthesized according to the procedure described in Example 1.

### 9.2 Synthesis of 7-(((((2R,3R,5R)-5-(4-amino-2-oxopyrimidin-1(2H)-yl)-4,4-difluoro-3-hydroxytetrahydrofuran-2-yl)methoxy)carbonyl)oxy)heptanoic acid (6):

To a DCM (10 mL) solution of 4 (1g, 4.6 mmol), 4-nitrophenyl chloroformate (2.79g, 13.8 mmol), DIPEA (2.38g, 18.4 mmol) and a catalytic amount of pyridine were added at 0°C and stirred for 5h at room temperature. Then the reaction mixture was concentrated *in vacuo.* The crude residue was dissolved in DMF (10 mL). To this solution, GMC hydrochloride (4.1g, 13.8 mmol) in DMF (5mL) and TEA (2 mL) were added and continued to stir for 24 h. The progress of the reaction was monitored by GC-MS analysis of the crude reaction mixture. After completion of reaction, excess diethyl ether (200 mL) was added to the reaction mixture. The yellowish oil thus obtained was separated and purified by flash chromatography using MeOH/CHCl₃ (5%, v/v) as eluent. Compound 6 was isolated as a sticky yellow liquid. (1.5g, Yield = 64.3%).

¹H NMR (DMSO-d₆): δ 10.5 (brs, 1H, -COOH), 7.63 (d, J = 7.5 Hz, 1H, -CH), 7.41 (brs, 2H, -NH₂), 6.20 (d, J = 7.5 Hz, 1H, -CH), 5.18 (brs, 1H, -CH), 3.71-3.55 (m, 5H, -CH₂ X2, -CHX1), 2.36 (brs, 2H, -CH₂), 1.23-1.17 (m, 18H, -CH₂ X 9).

¹³C NMR (DMSO-d6): 172.1, 166.0, 155.1, 154.9, 153.6, 123.5, 95.2, 95.0, 80.8, 69.1, 68.8, 33.6, 29.4, 29.3,29.2, 28.9, 28.8, 25.5, 25.4.

ESI-MS: cald for C₂₂H₃₃F₂N₃O₈, 504.2; found 527.0 (M +Na salt).

### 9.3 Synthesis of 1-((2R,3R,5R)-5-(4-amino-2-oxopyrimidin-1(2H)-yl)-4,4-difluoro-3-hydroxytetrahydrofuran-2-yl)-3,16,24-trioxo-20-(2-(5-(2-oxohexahydro-1H-thieno[3,4-d]imidazol-4-yl)pentanamido)ethyl)-2,4-dioxa-17,20,23-triazahentriacontan-31-yl 2, 3,4, 5-tetrachloro-6-(6-hydroxy-2, 4, 5, 7-tetraiodo-3-oxo-3H-xanthen-9-yl)benzoate (9):

EDCl, HCl (0.4g, 2.0 mmol), and DIPEA (0.45g, 3.5 mmol) were added to a solution of the acid 6 (0.34g, 0.6 mmol), compound 3 (0.25g, 0.6 mmol) and HOBt (0.27g, 2.0 mmol) in anhydrous DMF (50 mL) and stirred for 24 hrs at room temperature under nitrogen. The progress of the reaction was monitored by GC-MS analysis of the crude reaction mixture. After completion of reaction, RB-Octanoic Acid, 8 (prepared according to the literature procedure, 0.8g, 0.7 mmol) in 5 mL of DMF was added to the reaction mixture followed by a catalytic amount of DIPEA and continued to stir for 24 h at room temperature. After completion of reaction, excess diethyl ether (200 mL) was added to the reaction mixture and stirred for 30 min. The pink red precipitate thus obtained was filtered and was washed several times with cold diethyl ether (100 mL), ethyl acetate (100 mL), acetone-water mixture (10%, v/v, 100 mL) and finally with ethyl acetate-hexane mixture (50%, v/v, 100 ml) respectively to afford a pink red powder of compound 9 (0.63g, 45% yield).

¹H NMR (DMSO-d₆): δ 7.86 (s, 2H, Ar-CH), 7.73 (d, J = 7.0 Hz, 1H, CH), 7.39 (brs, 3H, NH X 3), 6.40-6.34 (m, 2H, NH X2), 6.03 (s, 1H, CH), 5.72 (d, J = 7.0 Hz, 1H, CH), 4.22 (s, 1H, CH), 4.05 (brs, 3H, CH₂, CH X2), 3.86 (brs, 1H, OH), 3.81-3.50 (m, 6H, CH X 2, CH₂X 2), 3.02-2.86 (m, 8H, CH₂X 4), 2.80-2.00 (m, 12H, CH₂ X 6), 1.52-0.81 (m, 32H, CH₂ X 16).

¹³C NMR (DMSO-d₆): 172.6, 171.6, 169.9, 168.9, 166.0, 163.2, 162.7, 155.1, 141.5, 141.1, 123.5, 98.9, 96.4, 95.0, 83.9, 80.8, 70.2, 69.1, 68.9, 68.7, 61.4, 59.6, 59.2, 54.2, 54.0, 48.9, 46.0, 38.0, 37.3, 36.1, 35.5, 31.1, 28.9, 28.6, 28.3, 25.7.

ESI-MS: cald for C₆₆H₇₉ Cl₄F₂I₄N₉O₁₅S, 1955.03; found 1956.5 (M +H).

## Claims

1. A microbubble-sonosensitiser complex for use in the treatment of metastatic disease via sonodynamic therapy, wherein said sonodynamic therapy comprises simultaneous, separate or sequential use of an immune checkpoint inhibitor; and further wherein said sonosensitiser is Rose Bengal and said immune checkpoint inhibitor is an inhibitor of PDL-1.

2. A complex for use as claimed in claim 1, wherein said microbubble-sonosensitiser complex comprises a microbubble attached to or otherwise associated with said sonosensitiser via a non-covalent linkage, e.g. via a biotin-avidin interaction.

3. A complex for use as claimed in claim 1 or claim 2, wherein said microbubble-sonosensitiser complex further comprises at least one chemotherapeutic agent, preferably wherein the chemotherapeutic agent is an anti-metabolite, e.g. 5-fluorouracil or gemcitabine.

4. A complex for use as claimed in claim 3, wherein said microbubble-sonosensitiser complex is attached to or otherwise associated with said at least one chemotherapeutic agent, preferably via a non-covalent linkage, e.g. via a biotin-avidin interaction, and/or wherein the microbubble comprises a shell having incorporated therein a chemotherapeutic agent.

5. A complex for use as claimed in claim 3 or claim 4, wherein the microbubble comprises a shell having incorporated therein an additional chemotherapeutic agent, preferably wherein said additional chemotherapeutic agent is an anti-microtubule agent, e.g. a taxol such as paclitaxel.

6. A complex for use as claimed in any one of claims 1 to 5, wherein said sonodynamic therapy further comprises simultaneous, separate or sequential use of a microbubble-chemotherapeutic agent complex, preferably wherein said microbubble-chemotherapeutic agent complex comprises a microbubble attached to or otherwise associated with at least one chemotherapeutic agent via a non-covalent linkage, e.g. via a biotin-avidin interaction, more preferably wherein said chemotherapeutic agent is an anti-metabolite, e.g. 5-fluorouracil or gemcitabine.

7. A complex for use as claimed in any one of the preceding claims, wherein the microbubble comprises a shell which retains a gas, preferably oxygen gas; and/or wherein the microbubble has a diameter in the range of from 0.1 to 100µm.

8. A complex for use as claimed in any one of the preceding claims, wherein the microbubble has a shell comprising one or more phospholipids, each optionally linked to one or more polymers, e.g. polyethylene glycol (PEG).

9. A complex for use as claimed in any one of the preceding claims, wherein said immune checkpoint inhibitor is selected from the group consisting of atezolizumab, avelumab, duravlumab, and any combination thereof.

10. A complex for use as claimed in any one of the preceding claims, in which said complex is contacted with cells or tissues of a subject (e.g. a human patient) and, either simultaneously or sequentially, said cells or tissues are subjected to irradiation with ultrasound.

11. A complex for use as claimed in any one of the preceding claims in the treatment of metastasis derived from breast, prostate or pancreatic cancer.

12. A complex for use as claimed in claim 11 in the treatment of metastatic pancreatic cancer.

13. A product comprising a microbubble-sonosensitiser complex as defined in any one of claims 1 to 8 and an immune checkpoint inhibitor as defined in claim 1 or claim 9 for simultaneous, separate or sequential use in the treatment of metastatic disease via sonodynamic therapy.

14. A pharmaceutical composition comprising a microbubble-sonosensitiser complex as defined in any one of claims 1 to 8 and an immune checkpoint inhibitor as defined in claim 1 or claim 9, together with at least one pharmaceutical carrier or excipient.

15. A composition as claimed in claim 14 for use in the treatment of metastatic disease via sonodynamic therapy.

## Patentansprüche

1. Mikroblasen-Sonosensitizerkomplex zur Verwendung bei der Behandlung einer metastatischen Krankheit über sonodynamische Therapie, wobei die sonodynamische Therapie gleichzeitige, getrennte oder aufeinanderfolgende Verwendung eines Immuncheckpoint-Inhibitors umfasst; und weiter wobei der Sonosensitizer Bengalrosa ist und der Immuncheckpoint-Inhibitor ein Inhibitor von PDL-1 ist.

2. Komplex zur Verwendung nach Anspruch 1, wobei der Mikroblasen-Sonosensitizerkomplex eine Mikroblase umfasst, die an dem Sonosensitizer über eine nichtkovalente Bindung, z. B. eine Biotin-Avidin-Interaktion, angehängt oder anderweitig damit verbunden ist.

3. Komplex zur Verwendung nach Anspruch 1 oder Anspruch 2, wobei der Mikroblasen-Sonosensitizerkomplex weiter mindestens ein Chemotherapeutikum umfasst, vorzugsweise wobei das Chemotherapeutikum ein Antimetabolit, z. B. 5-Fluorouracil oder Gemcitabin ist.

4. Komplex zur Verwendung nach Anspruch 3, wobei der Mikroblasen-Sonosensitizerkomplex an dem mindestens einen Chemotherapeutikum angehängt oder anderweitig damit verbunden ist, vorzugsweise über eine nichtkovalente Bindung, z. B. über eine Biotin-Avidin-Interaktion, und/oder wobei die Mikroblase eine Hülle umfasst, die darin integriert ein Chemotherapeutikum aufweist.

5. Komplex zur Verwendung nach Anspruch 3 oder Anspruch 4, wobei die Mikroblase eine Hülle umfasst, die darin integriert ein zusätzliches Chemotherapeutikum aufweist, vorzugsweise wobei das zusätzliche Chemotherapeutikum ein Antimikrotubuli-Wirkstoff, z. B. ein Taxol, wie Paclitaxel ist.

6. Komplex zur Verwendung nach einem der Ansprüche 1 bis 5, wobei die sonodynamische Therapie weiter gleichzeitige, separate oder aufeinanderfolgende Verwendung eines Mikroblasen-Chemotherapeutikumskomplexes umfasst, vorzugsweise wobei der Mikroblasen-Chemotherapeutikumskomplex eine Mikroblase umfasst, die an dem mindestens einen Chemotherapeutikum über eine nichtkovalente Bindung angehängt oder anderweitig damit verbunden ist, z. B. über eine Biotin-Avidin-Interaktion, besonders bevorzugt wobei das Chemotherapeutikum ein Antimetabolit ist, z. B. 5-Fluorouracil oder Gemcitabin.

7. Komplex zur Verwendung nach einem der vorstehenden Ansprüche, wobei die Mikroblase eine Hülle umfasst, die ein Gas, vorzugsweise Sauerstoffgas zurückhält; und/oder wobei die Mikroblase einen Durchmesser im Bereich von 0,1 bis 100 µm aufweist.

8. Komplex zur Verwendung nach einem der vorstehenden Ansprüche, wobei die Mikroblase eine Hülle aufweist, die ein oder mehrere Phospholipide umfasst, wobei jedes optional mit einem oder mehreren Polymeren, z. B. Polyethylenglykol (PEG) verbunden ist.

9. Komplex zur Verwendung nach einem der vorstehenden Ansprüche, wobei der Immuncheckpoint-Inhibitor ausgewählt aus der Gruppe bestehend aus Atezolizumab, Avelumab, Duravlumab und einer Kombination daraus.

10. Komplex zur Verwendung nach einem der vorstehenden Ansprüche, in dem der Komplex mit Zellen oder Geweben eines Subjekts (z. B. eines menschlichen Patienten) in Kontakt gebracht wird und die Zellen oder Gewebe entweder gleichzeitig oder aufeinanderfolgend Bestrahlung mit Ultraschall unterzogen werden.

11. Komplex zur Verwendung nach einem der vorstehenden Ansprüche bei der Behandlung von Metastasen infolge von Brust-, Prostata- oder Bauchspeicheldrüsenkrebs.

12. Komplex zur Verwendung nach Anspruch 11 bei der Behandlung von metastatischem Bauchspeicheldrüsenkrebs.

13. Produkt, das einen Mikroblasen-Sonosensitizerkomplex nach einem der Anspruchs 1 bis 8 und Immuncheckpoint-Inhibitor nach Anspruch 1 oder Anspruch 9 zur gleichzeitigen, separaten oder aufeinanderfolgenden Verwendung bei der Behandlung einer metastatischen Krankheit über sonodynamische Therapie umfasst.

14. Pharmazeutischen Zusammensetzung, die einen Mikroblasen-Sonosensitizerkomplex nach einem der Anspruchs 1 bis 8 und einen Immuncheckpoint-Inhibitor nach Anspruch 1 oder Anspruch 9 umfasst, zusammen mit mindestens einem pharmazeutischen Träger oder Hilfsstoff.

15. Zusammensetzung nach Anspruch 14 zur Verwendung bei der Behandlung einer metastatischen Krankheit über sonodynamische Therapie.

## Revendications

1. Complexe à microbulles sono-sensibilisateur pour être utilisé dans le traitement de maladies métastatiques par thérapie sonodynamique, dans lequel ladite thérapie sonodynamique comprend l'utilisation simultanée, séparée ou séquentielle d'un inhibiteur de point de contrôle immunitaire ; et en outre dans lequel ledit sono-sensibilisateur est le Rose de bengale et ledit inhibiteur de point de contrôle immunitaire est un inhibiteur de PDL-1.

2. Complexe pour être utilisé selon la revendication 1, dans lequel ledit complexe à microbulles sono-sensibilisateur comprend une microbulle fixée à ou autrement associée audit sono-sensibilisateur par une liaison non covalente, par exemple par une interaction biotine-avidine.

3. Complexe pour être utilisé selon la revendication 1 ou la revendication 2, dans lequel ledit complexe à microbulles sono-sensibilisateur comprend en outre au moins un agent chimiothérapeutique, de préférence dans lequel l'agent chimiothérapeutique est un antimétabolite, par exemple le 5-fluorouracile ou la gemcitabine.

4. Complexe pour être utilisé selon la revendication 3, dans lequel ledit complexe à microbulles sono-sensibilisateur est fixé à ou autrement associé audit au moins un agent chimiothérapeutique, de préférence par l'intermédiaire d'une liaison non covalente, par exemple par l'intermédiaire d'une interaction biotine-avidine, et/ou dans lequel la microbulle comprend une enveloppe dans laquelle est incorporé un agent chimiothérapeutique.

5. Complexe pour être utilisé selon la revendication 3 ou la revendication 4, dans lequel la microbulle comprend une enveloppe dans laquelle est incorporé un agent chimiothérapeutique supplémentaire, de préférence dans lequel ledit agent chimiothérapeutique supplémentaire est un agent antimicrotubulaire, par exemple un taxol tel que le paclitaxel.

6. Complexe pour être utilisé selon l'une quelconque des revendications 1 à 5, dans lequel ladite thérapie sonodynamique comprend en outre l'utilisation simultanée, séparée ou séquentielle d'un complexe à microbulles agent chimiothérapeutique, de préférence dans lequel ledit complexe à microbulles agent chimiothérapeutique comprend une microbulle fixée à ou autrement associée à au moins un agent chimiothérapeutique via une liaison non covalente, par exemple via une interaction biotine-avidine, de préférence dans lequel ledit agent chimiothérapeutique est un antimétabolite, par exemple le 5-fluorouracile ou la gemcitabine.

7. Complexe pour être utilisé selon l'une quelconque des revendications précédentes, dans lequel la microbulle comprend une enveloppe qui retient un gaz, de préférence de l'oxygène gazeux ; et/ou dans lequel la microbulle présente un diamètre compris dans la plage de 0,1 à 100 µm.

8. Complexe pour être utilisé selon l'une quelconque des revendications précédentes, dans lequel la microbulle présente une enveloppe comprenant un ou plusieurs phospholipides, chacun étant optionnellement lié à un ou plusieurs polymères, par exemple le polyéthylène glycol (PEG).

9. Complexe pour être utilisé selon l'une quelconque des revendications précédentes, dans lequel ledit inhibiteur de point de contrôle immunitaire est choisi dans le groupe consistant en l'atezolizumab, l'avélumab, le duravlumab et toute combinaison de ceux-ci.

10. Complexe pour être utilisé selon l'une quelconque des revendications précédentes, dans lequel ledit complexe est mis en contact avec des cellules ou des tissus d'un sujet (par exemple un patient humain) et, soit simultanément soit séquentiellement, lesdites cellules ou tissus sont soumis à une irradiation par ultrasons.

11. Complexe destiné à être utilisé selon l'une quelconque des revendications précédentes dans le traitement des métastases dérivées d'un cancer du sein, de la prostate ou du pancréas.

12. Complexe destiné à être utilisé selon la revendication 11 dans le traitement du cancer métastatique du pancréas.

13. Produit comprenant un complexe à microbulles sono-sensibilisateur tel que défini dans l'une quelconque des revendications 1 à 8 et un inhibiteur de point de contrôle immunitaire tel que défini dans la revendication 1 ou la revendication 9, pour être utilisé simultanément, séparément ou séquentiellement dans le traitement d'une maladie métastatique par thérapie sonodynamique.

14. Composition pharmaceutique comprenant un complexe à microbulles sono-sensibilisateur tel que défini dans l'une quelconque des revendications 1 à 8 et un inhibiteur de point de contrôle immunitaire tel que défini dans la revendication 1 ou la revendication 9, ainsi qu'au moins un excipient ou un vecteur pharmaceutique.

15. Composition selon la revendication 14 destinée à être utilisée dans le traitement de maladies métastatiques par thérapie sonodynamique.
